# EUROPÄISCHE PATENTSCHRIFT

(11) **EP 2 802 580 B1**
(45) Veröffentlichungstag und Bekanntmachung des Hinweises auf die Patenterteilung: **13.07.2016**
(21) Anmeldenummer: 13700278.8
(22) Anmeldetag: 08.01.2013
(51) Int. Cl.: C07D 471/04, A61K 31/53, A61P 9/00, A61K 45/06

(54) **SUBSTITUIERTE TRIAZINE DERIVATE UND IHRE VERWENDUNG ALS STIMULATOREN DER LÖSLICHEN GUANYLATCYCLASE**
SUBSTITUTED TRIAZINE DERIVATIVES AND THEIR USE AS STIMULATORS OF SOLUBLE GUANYLATE CYCLASE
DÉRIVÉS DE LA TRIAZINE SUBSTITUÉE ET LEUR UTILISATION EN TANT QUE STIMULATEURS DE LA GUANYLATE CYCLASE SOLUBLE

(30) Priorität: 11.01.2012 DE 102012200360
(43) Veröffentlichungstag der Anmeldung: 19.11.2014
(73) Patentinhaber: Bayer Intellectual Property GmbH, 40789 Monheim (DE)
(72) Erfinder: FOLLMANN, Markus, 50859 Köln (DE); STASCH, Johannes-Peter, 42651 Solingen (DE); REDLICH, Gorden, 44799 Bochum (DE); GRIEBENOW, Nils, 41541 Dormagen (Zons) (DE); WUNDER, Frank, 42117 Wuppertal (DE); LANG, Dieter, 42553 Velbert (DE)
(74) Vertreter: BIP Patents
(86) Internationale Anmeldenummer: PCT/EP2013/050179
(87) Internationale Veröffentlichungsnummer: WO 2013/104597

(56) Entgegenhaltungen:
- WO-A1-00/06569
- WO-A1-2011/115804
- WO-A2-2006/081230

## Beschreibung

Die vorliegende Anmeldung betrifft neue substituierte Triazine, Verfahren zu ihrer Herstellung, ihre Verwendung allein oder in Kombinationen zur Behandlung und/oder Prophylaxe von Krankheiten sowie ihre Verwendung zur Herstellung von Arzneimitteln zur Behandlung und/oder Prophylaxe von Krankheiten, insbesondere zur Behandlung und/oder Prophylaxe von Herz-Kreislauf-Erkrankungen.

Eines der wichtigsten zellulären Übertragungssysteme in Säugerzellen ist das cyclische Guanosinmonophosphat (cGMP). Zusammen mit Stickstoffmonoxid (NO), das aus dem Endothel freigesetzt wird und hormonelle und mechanische Signale überträgt, bildet es das NO/cGMP-System. Die Guanylatcyclasen katalysieren die Biosynthese von cGMP aus Guanosintriphosphat (GTP). Die bisher bekannten Vertreter dieser Familie lassen sich sowohl nach strukturellen Merkmalen als auch nach der Art der Liganden in zwei Gruppen aufteilen: Die partikulären, durch natriuretische Peptide stimulierbaren Guanylatcyclasen und die löslichen, durch NO stimulierbaren Guanylatcyclasen. Die löslichen Guanylatcyclasen bestehen aus zwei Untereinheiten und enthalten höchstwahrscheinlich ein Häm pro Heterodimer, das ein Teil des regulatorischen Zentrums ist. Dieses hat eine zentrale Bedeutung für den Aktivierungsmechanismus. NO kann an das Eisenatom des Häms binden und so die Aktivität des Enzyms deutlich erhöhen. Hämfreie Präparationen lassen sich hingegen nicht durch NO stimulieren. Auch Kohlenmonoxid (CO) ist in der Lage, an das Eisen-Zentralatom des Häms zu binden, wobei die Stimulierung durch CO deutlich geringer ist als die durch NO.

Durch die Bildung von cGMP und der daraus resultierenden Regulation von Phosphodiesterasen, Innenkanälen und Proteinkinasen spielt die Guanylatcyclase eine entscheidende Rolle bei unterschiedlichen physiologischen Prozessen, insbesondere bei der Relaxation und Proliferation glatter Muskelzellen, der Plättchenaggregation und -adhäsion, der neuronalen Signalübertragung sowie bei Erkrankungen, welche auf einer Störung der vorstehend genannten Vorgänge beruhen. Unter pathophysiologischen Bedingungen kann das NO/cGMP-System supprimiert sein, was zum Beispiel zu Bluthochdruck, einer Plättchenaktivierung, einer vermehrten Zellproliferation, endothelialer Dysfunktion, Arteriosklerose, Angina pectoris, Herzinsuffizienz, Myokardinfarkt, Thrombosen, Schlaganfall und sexueller Dysfunktion führen kann.

Eine auf die Beeinflussung des cGMP-Signalweges in Organismen abzielende NO-unabhängige Behandlungsmöglichkeit für derartige Erkrankungen ist aufgrund der zu erwartenden hohen Effizienz und geringen Nebenwirkungen ein vielversprechender Ansatz.

Zur therapeutischen Stimulation der löslichen Guanylatcyclase wurden bisher ausschließlich Verbindungen wie organische Nitrate verwendet, deren Wirkung auf NO beruht. Dieses wird durch Biokonversion gebildet und aktiviert die lösliche Guanylatcyclase durch Angriff am Eisen-Zentralatom des Häms. Neben den Nebenwirkungen gehört die Toleranzentwicklung zu den entscheidenden Nachteilen dieser Behandlungsweise.

In den letzten Jahren wurden einige Substanzen beschrieben, die die lösliche Guanylatcyclase direkt, d.h. ohne vorherige Freisetzung von NO stimulieren, wie beispielsweise 3-(5'-Hydroxy-methyl-2'-furyl)-1-benzylindazol [YC-1; Wu et al., Blood 84 (1994), 4226; Mülsch et al., Brit. J. Pharmacol. 120 (1997), 681], Fettsäuren [Goldberg et al., J. Biol. Chem. 252 (1977), 1279], Diphenyliodonium-hexafluorophosphat [Pettibone et al., Eur. J. Pharmacol. 116 (1985), 307], Isoliquiritigenin [Yu et al., Brit. J. Pharmacol. 114 (1995), 1587] sowie verschiedene substituierte Pyrazol-Derivate (WO 98/16223).

Als Stimulatoren der löslichen Guanylatcyclase werden in WO 00/06568 und WO 00/06569 annellierte Pyrazol-Derivate und in WO 03/095451 Carbamat-substitutierte 3-Pyrimidinyl-Pyrazolopyridine offenbart. 3-Pyrimidinyl-Pyrazolopyridine mit Phenylamid-Substituenten werden in E. M. Becker et al., BMC Pharmacology 1 (13), 2001 beschrieben. WO 2004/009590 beschreibt Pyrazolopyridine mit substituierten 4-Aminopyrimidinen zur Behandlung von ZNS-Erkrankungen. WO 2010/065275 offenbart substituierte Pyrrolo- und Dihydropyridopyrimidine als sGC Aktivatoren.

Aufgabe der vorliegenden Erfindung war die Bereitstellung neuer Substanzen, die als Stimulatoren der löslichen Guanylatcyclase wirken und ein gleiches oder verbessertes therapeutisches Profil gegenüber den aus dem Stand der Technik bekannten Verbindungen aufweisen, wie beispielsweise hinsichtlich ihrer *in-vivo* Eigenschaften, wie beispielsweise ihrem pharmakokinetischem und pharmakodynamischem Verhalten und/oder ihres Metabolismus-Profils und/oder ihrer Dosis-Wirkungsbeziehung.

Gegenstand der Offenbarung sind Verbindungen der allgemeinen Formel (I) 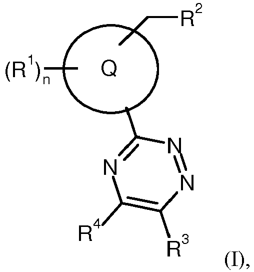 in welcher
der Ring Q für 8- oder 9-gliedriges Heteroaryl steht,
- R¹: für Halogen, Cyano, Difluormethyl, Trifluormethyl, (C₁-C₄)-Alkyl, Hydroxy, Oxo oder (C₁-C₄)-Alkoxy steht,
- n: für eine Zahl 0, 1 oder 2 steht,
- R²: für Trifluormethyl, (C₁-C₆)-Alkyl, (C₃-C₈)-Cycloalkyl, Phenyl oder 5- oder 6-gliedriges Heteroaryl steht, wobei (C₁-C₆)-Alkyl mit einem Substituenten ausgewählt aus der Gruppe Difluormethyl und Trifluormethyl substituiert ist, wobei (C₁-C₆)-Alkyl mit 1 bis 3 Substituenten Fluor substituiert sein kann, wobei (C₃-C₈)-Cycloalkyl mit 1 oder 2 Substituenten unabhängig voneinander ausgewählt aus der Gruppe Fluor, Methyl und Methoxy substituiert sein kann, wobei Phenyl mit 1 bis 3 Substituenten Fluor substituiert ist, wobei Phenyl mit 1 oder 2 Substituenten unabhängig voneinander ausgewählt aus der Gruppe Methyl und Methoxy substituiert sein kann, und wobei 5- und 6-gliedriges Heteroaryl mit 1 oder 2 Substituenten unabhängig voneinander ausgewählt aus der Gruppe Fluor und Methyl substituiert sein können,
- R³: für Difluormethyl, Trifluormethyl, (C₁-C₆)-Alkyl, (C₃-C₇)-Cycloalkyl, (C₁-C₆)-Alkylsulfonylamino, (C₁-C₆)-Alkoxycarbonylamino, Phenyl oder 5- oder 6-gliedriges Heteroaryl steht, wobei (C₁-C₆)-Alkyl, Phenyl und 5- oder 6-gliedriges Heteroaryl mit 1 bis 3 Substituenten unabhängig voneinander ausgewählt aus der Gruppe Halogen, Trifluormethyl, (C₁-C₄)-Alkyl, (C₃-C₇)-Cycloalkyl, Difluormethoxy, Trifluormethoxy und (C₁-C₆)-Alkoxy substituiert sein können,
- R⁴: für Hydroxy oder Amino steht,
sowie ihre *N*-Oxide, Salze, Solvate, Salze der *N*-Oxide und Solvate der *N*-Oxide und Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Ameisensäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomere und Diastereomere und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler bzw. chiraler Phase.

Sofern die erfindungsgemäßen Verbindungen in tautomeren Formen vorkommen können, umfasst die vorliegende Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie ²H (Deuterium), ³H (Tritium), ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁹I und ¹³¹I. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit ³H- oder ¹⁴C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise eine Verlängerung der Halbwertszeit im Körper oder eine Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach den dem Fachmann bekannten Verfahren hergestellt werden, so beispielsweise nach den weiter unten beschriebenen Methoden und den bei den Ausführungsbeispielen wiedergegebenen Vorschriften, indem entsprechende isotopische Modifikationen der jeweiligen Reagentien und/oder Ausgangsverbindungen eingesetzt werden.

Außerdem werden auch Prodrugs der erfindungsgemäßen Verbindungen beschrieben.

Der Begriff "Prodrugs" bezeichnet hierbei Verbindungen, welche selbst biologisch aktiv oder inaktiv sein können, jedoch während ihrer Verweilzeit im Körper zu erfindungsgemäßen Verbindungen umgesetzt werden (beispielsweise metabolisch oder hydrolytisch).

Im Rahmen der vorliegenden Erfindung haben die Substituenten, soweit nicht anders spezifiziert, die folgende Bedeutung:
Alkyl steht im Rahmen der Erfindung für einen linearen oder verzweigten Alkylrest mit der jeweils angegebenen Anzahl an Kohlenstoffatomen. Beispielhaft und vorzugsweise seien genannt: Methyl, Ethyl, n-Propyl, Isopropyl, n-Butyl, iso-Butyl, 1-Methylpropyl, tert.-Butyl, n-Pentyl, iso-Pentyl, 1-Ethylpropyl, 1-Methylbutyl, 2-Methylbutyl, 3-Methylbutyl, n-Hexyl, 1-Methylpentyl, 2-Methylpentyl, 3-Methylpentyl, 4-Methylpentyl, 3,3-Dimethylbutyl, 1-Ethylbutyl und 2-Ethylbutyl.

5- bis 7-gliedriger gesättigter oder teilweise ungesättigter Carbocyclus steht in Rahmen der Erfindung für einen gesättigten oder teilweise ungesättigten cyclischen Alkylrest mit der jeweils angegebenen Anzahl an Kohlenstoffatomen. Beispielhaft und vorzugsweise seien genannt: Cyclopentyl, Cyclohexyl, Cycloheptyl, Cyclopentenyl, Cyclohexenyl und Cycloheptenyl.

Cycloalkyl bzw. Carbocyclus steht in Rahmen der Erfindung für einen monocyclischen, gesättigten Alkylrest mit der jeweils angegebenen Anzahl an Kohlenstoffatomen. Beispielhaft und vorzugsweise seien genannt: Cyclopropyl, Cyclobutyl, Cyclopentyl, Cyclohexyl und Cycloheptyl.

Alkoxy steht im Rahmen der Erfindung für einen linearen oder verzweigten Alkoxyrest mit 1 bis 6 bzw. 1 bis 4 Kohlenstoffatomen. Beispielhaft seien genannt: Methoxy, Ethoxy, n-Propoxy, Isopropoxy, 1-Methylpropoxy, n-Butoxy, iso-Butoxy, tert.-Butoxy, n-Pentoxy, iso-Pentoxy, 1-Ethylpropoxy, 1-Methylbutoxy, 2-Methylbutoxy, 3-Methylbutoxy und n-Hexoxy. Bevorzugt ist ein linearer oder verzweigter Alkoxyrest mit 1 bis 4 Kohlenstoffatomen. Beispielhaft und vorzugsweise seien genannt: Methoxy, Ethoxy, n-Propoxy, Isopropoxy, 1-Methylpropoxy, n-Butoxy, iso-Butoxy, tert.-Butoxy.

Alkoxycarbonylamino steht im Rahmen der Erfindung für eine Amino-Gruppe mit einem linearen oder verzweigten Alkoxycarbonyl-Substituenten, der 1 bis 4 Kohlenstoffatome in der Alkylkette aufweist und über die Carbonylgruppe mit dem N-Atom verknüpft ist. Beispielhaft und vorzugsweise seien genannt: Methoxycarbonylamino, Ethoxycarbonylamino, Propoxycarbonylamino, n-Butoxycarbonylamino, iso-Butoxycarbonylamino und tert.-Butoxycarbonylamino.

Alkylsulfonylamino steht im Rahmen der Erfindung für eine Amino-Gruppe mit einem linearen oder verzweigten Alkylsulfonyl-Substituenten, der 1 bis 6 Kohlenstoffatome aufweist und über die Sulfonylgruppe mit dem N-Atom verknüpft ist. Beispielhaft und vorzugsweise seien genannt: Methylsulfonylamino, Ethylsulfonylamino, *n*-Propylsulfonylamino, Isopropylsulfonylamino, *n-*Butylsulfonylamino, *tert*.-Butylsulfonylamino, *n*-Pentylsulfonylamino und *n*-Hexylsulfonylamino.

5- bis 7-gliedrigen gesättigter oder teilweise ungesättigter Heterocyclus steht im Rahmen der Erfindung für einen gesättigten oder teilweise ungesättigten Heterocyclus mit insgesamt 5 bis 7 Ringatomen, der ein Ring-Heteroatome aus der Reihe N, O, S, SO und/oder SO₂ enthält. Beispielhaft seien genannt: Pyrrolidinyl, Tetrahydrofuranyl, Piperidinyl, Tetrahydropyranyl, Dihydropyrrolyl, Dihydropyridyl.

5- oder 6-gliedriges Heteroaryl steht im Rahmen der Erfindung für einen monocyclischen aromatischen Heterocyclus (Heteroaromaten) mit insgesamt 5 oder 6 Ringatomen, der bis zu drei gleiche oder verschiedene Ring-Heteroatome aus der Reihe N, O und/oder S enthält und über ein Ring-Kohlenstoffatom oder gegebenenfalls über ein Ring-Stickstoffatom verknüpft ist. Beispielhaft und vorzugsweise seien genannt: Furyl, Pyrrolyl, Thienyl, Pyrazolyl, Imidazolyl, Thiazolyl, Oxazolyl, Isoxazolyl, Isothiazolyl, Triazolyl, Oxadiazolyl, Thiadiazolyl, Pyridyl, Pyrimidinyl, Pyridazinyl, Pyrazinyl und Triazinyl. Bevorzugt sind: Pyridyl, Pyrimidinyl, Pyridazinyl, Pyrazinyl und Triazinyl.

8- oder 9-gliedriges Heteroaryl steht im Rahmen der Erfindung für einen bicyclischen aromatischen oder teilweise ungesättigten Heterocyclus mit insgesamt 8 oder 9 Ringatomen, der mindestens zwei Stickstoffatome und bis zu zwei weitere, gleiche oder verschiedene Ring-Heteroatome aus der Reihe N, O und/oder S enthält. Beispielhaft seien genannt: Dihydrothienopyrazolyl, Thienopyrazolyl, Pyrazolopyrazolyl, Imidazothiazolyl, Tetrahydrocyclopentapyrazolyl, Dihydrocyclopentapyrazolyl, Tetrahydroindazolyl, Dihydroindazolyl, Indazolyl, Pyrazolopyridinyl, Tetrahydropyrazolopyridinyl, Pyrazolopyrimidinyl, Imidazopyridinyl und Imidazopyridazinyl.

Halogen steht im Rahmen der Erfindung für Fluor, Chlor, Brom und Iod. Bevorzugt sind Brom und Iod.

Eine Oxo-Gruppe steht im Rahmen der Erfindung für ein Sauerstoffatom, das über eine Doppelbindung an ein Kohlenstoffatom gebunden ist.

In der Formel der Gruppe, für die Q stehen kann, steht der Endpunkt der Linie, an dem das Zeichen * und ** steht, nicht für ein Kohlenstoffatom beziehungsweise eine CH₂-Gruppe, sondern ist Bestandteil der Bindung zu dem jeweils bezeichneten Atom, an das Q gebunden ist.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt.

Im Sinne der vorliegenden Erfindung umfasst der Begriff "Behandlung" oder "behandeln" ein Hemmen, Verzögern, Aufhalten, Lindern, Abschwächen, Einschränken, Verringern, Unterdrücken, Zurückdrängen oder Heilen einer Krankheit, eines Leidens, einer Erkrankung, einer Verletzung oder einer gesundheitlichen Störung, der Entfaltung, des Verlaufs oder des Fortschreitens solcher Zustände und/oder der Symptome solcher Zustände. Der Begriff "Therapie" wird hierbei als synonym mit dem Begriff "Behandlung" verstanden.

Die Begriffe "Prävention", "Prophylaxe" oder "Vorbeugung" werden im Rahmen der vorliegenden Erfindung synonym verwendet und bezeichnen das Vermeiden oder Vermindern des Risikos, eine Krankheit, ein Leiden, eine Erkrankung, eine Verletzung oder eine gesundheitliche Störung, eine Entfaltung oder ein Fortschreiten solcher Zustände und/oder die Symptome solcher Zustände zu bekommen, zu erfahren, zu erleiden oder zu haben.

Die Behandlung oder die Prävention einer Krankheit, eines Leidens, einer Erkrankung, einer Verletzung oder einer gesundheitlichen Störung können teilweise oder vollständig erfolgen.

Offenbarungsgegenstand sind Verbindungen der Formel (I), in welcher
der Ring Q für eine Gruppe der Formel 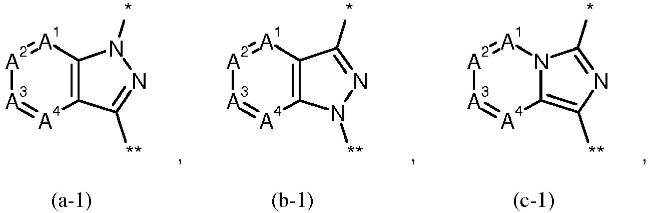 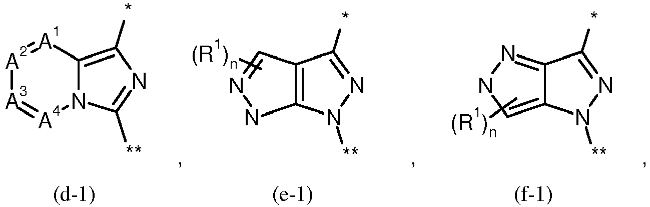 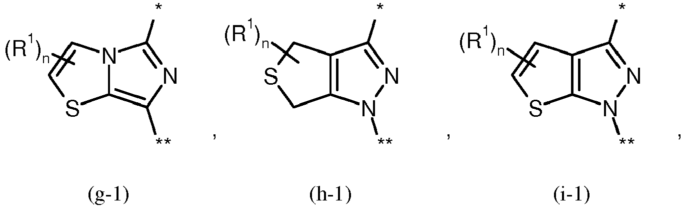 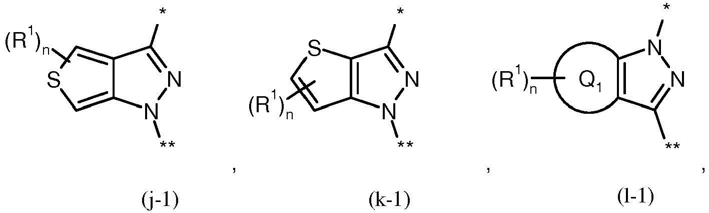 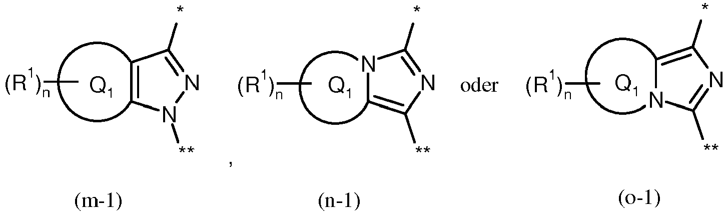 steht, wobei
- *: für die Anknüpfungsstelle an -CH₂-R² steht,
- **: für die Anknüpfungsstelle an den Triazinring steht,
- der Ring Q₁: zusammen mit den Atomen, an die er gebunden ist, einen 5- bis 7-gliedrigen gesättigten oder teilweise ungesättigten Carbocyclus oder einen 5- bis 7-gliedrigen gesättigten oder teilweise ungesättigten Heterocyclus bildet,
- R¹: für Fluor, Chlor oder Methyl steht,
- n: für eine Zahl 0, 1 oder 2 steht,
- A¹, A², A³ und A⁴: unabhängig voneinander jeweils für N, CH oder CR¹ stehen,
mit der Maßgabe, dass maximal zwei der Gruppen A¹, A², A³ und A⁴ für N stehen,
- R²: für Trifluormethyl, 2,2,2-Trifluorethyl, 3,3,3-Trifluorprop-1-yl, 2,2,3,3,3-Pentafluorprop-1-yl, Cyclopropyl, Cyclobutyl, Cyclopentyl, Cyclohexyl, Phenyl, Pyridyl, Pyrimidinyl, Pyrazinyl oder Pyridazinyl steht, wobei Phenyl mit 1 bis 3 Substituenten Fluor substituiert ist, und wobei Cyclopropyl, Cyclobutyl, Cyclopentyl, Cyclohexyl, Pyridyl, Pyrimidinyl, Pyrazinyl und Pyridazinyl mit 1 oder 2 Substituenten Fluor substituiert sein können,
- R³: für Difluormethyl, Trifluormethyl, (C₁-C₆)-Alkyl, Cyclopropyl, Cyclobutyl, Cyclopentyl, Methylsulfonylamino, Methoxycarbonylamino, Phenyl, Pyrazolyl, Oxazolyl oder Pyridyl steht, wobei (C₁-C₆)-Alkyl mit 1 bis 3 Substituenten unabhängig voneinander ausgewählt aus der Gruppe Fluor, Trifluormethyl, Cyclopropyl, Cyclobutyl, Cyclopentyl, Difluormethoxy, Trifluormethoxy, Methoxy und Ethoxy substituiert sein können, und wobei Phenyl, Pyrazolyl, Oxazolyl und Pyridyl mit 1 oder 2 Substituenten unabhängig voneinander ausgewählt aus der Gruppe Fluor, Chlor, Difluormethyl, Trifluormethyl, Methyl, Ethyl, Cyclopropyl, Cyclobutyl, Cyclopentyl, Trifluormethoxy, Methoxy und Ethoxy substituiert sein können,
- R⁴: für Hydroxy oder Amino steht,
sowie ihre Salze, Solvate und Solvate der Salze.

Gegenstand der vorliegenden Erfindung sind Verbindungen der Formel (I), in welcher der Ring Q für eine Gruppe der Formel 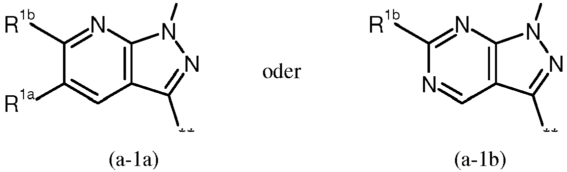 steht, wobei
- *: für die Anknüpfungsstelle an -CH₂-R² steht,

- **: für die Anknüpfungsstelle an den Triazinring steht,
- R^{1a}: für Wasserstoff oder Fluor steht,
- R^{1b}: für Wasserstoff oder Methyl steht,
- R²: für 3,3,3-Trifluorprop-1-yl, 2,2,3,3-Tetrafluorprop-1-yl, 2,2,3,3,3-Pentafluorprop-1-yl, Phenyl oder Pyridyl steht, wobei Phenyl mit 1 bis 3 Substituenten Fluor substituiert ist, und wobei Pyridyl mit 1 Substituenten Fluor substituiert sein kann,
- R³: für Difluormethyl, Trifluormethyl, (C₁-C₆)-Alkyl, Cyclopropyl, Cyclobutyl, Cyclopentyl, Methylsulfonylamino, Methoxycarbonylamino, Phenyl, Pyrazolyl, Oxazolyl oder Pyridyl steht, wobei (C₁-C₆)-Alkyl mit 1 bis 3 Substituenten unabhängig voneinander ausgewählt aus der Gruppe Fluor, Trifluormethyl, Cyclopropyl, Cyclobutyl, Cyclopentyl, Difluormethoxy, Trifluormethoxy, Methoxy und Ethoxy substituiert sein können, und wobei Phenyl, Pyrazolyl, Oxazolyl und Pyridyl mit 1 oder 2 Substituenten unabhängig voneinander ausgewählt aus der Gruppe Fluor, Chlor, Difluormethyl, Trifluormethyl, Methyl, Ethyl, Cyclopropyl, Cyclobutyl, Cyclopentyl, Trifluormethoxy, Methoxy und Ethoxy substituiert sein können,
- R⁴: für Hydroxy oder Amino steht,
sowie ihre Salze, Solvate und Solvate der Salze.

Bevorzugt sind im Rahmen der vorliegenden Erfindung auch Verbindungen der Formel (I), in welcher
der Ring Q für eine Gruppe der Formel 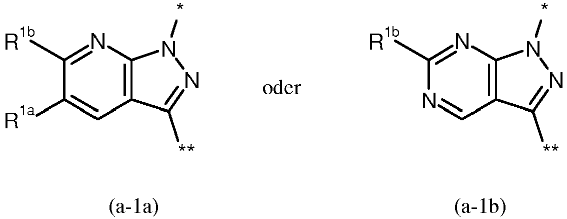 steht, wobei
- *: für die Anknüpfungsstelle an -CH₂-R² steht,
- **: für die Anknüpfungsstelle an den Triazinring steht,
- R^{1a}: für Wasserstoff oder Fluor steht,
- R^{1b}: für Wasserstoff oder Methyl steht,
- R^{1b}: für Wasserstoff oder Methyl steht,
sowie ihre Salze, Solvate und Solvate der Salze.

Bevorzugt sind im Rahmen der vorliegenden Erfindung auch Verbindungen der Formel (I), in welcher
- R²: für 2-Fluorphenyl, 2,3-Difluorphenyl oder 2,3,6-Trifluorphenyl steht,
sowie ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Besonders bevorzugt sind Kombinationen von zwei oder mehreren der oben genannten Vorzugsbereiche.

Weiterer Gegenstand der Erfindung ist ein Verfahren zur Herstellung der erfindungsgemäßen Verbindungen der Formel (I) dadurch gekennzeichnet, dass man
[A] eine Verbindung der Formel (II) 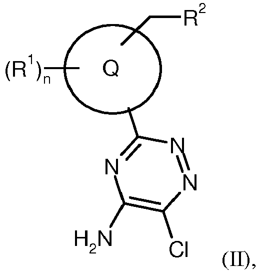 in welcher n, Q, R¹ und R² jeweils die oben genannten Bedeutungen haben, in einem inerten Lösungsmittel in Gegenwart eines geeigneten Übergangsmetallkatalysators mit einer Verbindung der Formel (III) 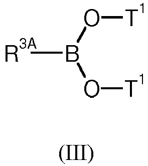 in welcher
   - R^{3A}: für Phenyl oder 5- oder 6-gliedriges Heteroaryl steht, wobei Phenyl und 5- oder 6-gliedriges Heteroaryl mit 1 bis 3 Substituenten unabhängig voneinander ausgewählt aus der Gruppe Halogen, Trifluormethyl, (C₁-C₄)-Alkyl, (C₃-C₇)-Cycloalkyl, Difluormethoxy, Trifluormethoxy und (C₁-C₆)-Alkoxy substituiert sein können,
   - und T¹: für Wasserstoff oder (C₁-C₄)-Alkyl steht, oder beide Reste R¹¹ zusanmlen eine -C(CH₃)₂-C(CH₃)₂-Brücke bilden,
   zu einer Verbindung der Formel (I-A) 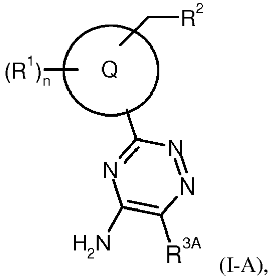 in welcher n, Q, R¹, R² und R^{3A} jeweils die oben angegebenen Bedeutungen haben,
   umsetzt,
   oder
[B] eine Verbindung der Formel (IV) 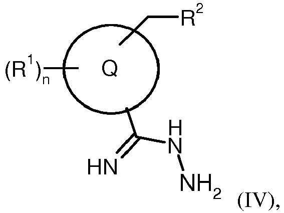 in welcher n, Q, R¹ und R² jeweils die oben angegebenen Bedeutungen haben, in einem inerten Lösungsmittel mit einer Verbindung der Formel (V) 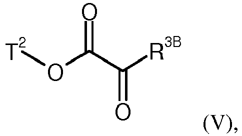 in welcher
   - R^{3B}: für Difluormethyl, Trifluormethyl, (C₁-C₆)-Alkyl oder (C₃-C₇)-Cycloalkyl steht, wobei (C₁-C₆)-Alkyl mit 1 bis 3 Substituenten unabhängig voneinander ausgewählt aus der Gruppe Halogen, Trifluormethyl, (C₁-C₄)-Alkyl, (C₃-C₇)-Cycloalkyl, Difluormethoxy, Trifluormethoxy und (C₁-C₆)-Alkoxy substituiert sein kann,
   - und T²: für (C₁-C₄)-Alkyl steht,
   zu einer Verbindung der Formel (I-B) 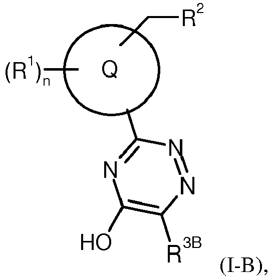 in welcher n, Q, R¹, R² und R^{3B} jeweils die oben angegebenen Bedeutungen haben,
   umsetzt,
   oder
[C] eine Verbindung der Formel (I-B) mit Phosphorylchlorid in eine Verbindung der Formel (VI) 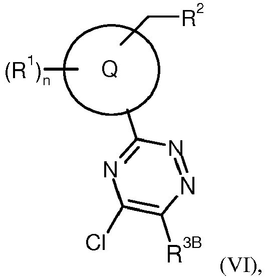 in welcher n, Q, R¹, R² und R^{3B} jeweils die oben angegebenen Bedeutungen haben, überführt, und diese direkt mit Ammoniak zu einer Verbindung der Formel (I-C) 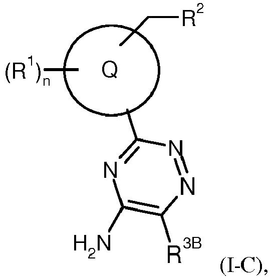 in welcher n, Q, R¹, R² und R^{3B} jeweils die oben angegebenen Bedeutungen haben,
   umsetzt,
   und gegebenenfalls die resultierenden Verbindungen der Formel (I-A), (I-B) und (I-C) gegebenenfalls mit den entsprechenden (i) Lösungsmitteln und/oder (ii) Säuren oder Basen in ihre Solvate, Salze und/oder Solvate der Salze überführt.

Der Verfahrensschritt (II)+ (III) → (I-A) erfolgt in einem unter den Reaktionsbedingungen inerten Lösungsmittel. Geeignete Lösungsmittel sind beispielsweise Ether wie Diethylether, Dioxan, Tetrahydrofuran, Glykoldimethylether oder Diethylenglykoldimethylether, oder anderen Lösungsmitten wie Dimethylformamid (DMF), Dimethylsulfoxid (DMSO), N.N'-Dimethylpropylenharnstoff (DMPU), *N*-Methylpyrrolidon (NMP), Pyridin, Acetonitril oder auch Wasser. Ebenso ist es möglich, Gemische der genannten Lösungsmittel einzusetzen. Bevorzugt ist Acetonitril.

Gegebenenfalls kann die Umsetzung (II)+ (III) → (I-A) in Gegenwart eines geeigneten Palladium- und/oder Kupferkatalysators erfolgen. Als Palladium-Katalysator ist beispielsweise Palladium auf Aktivkohle, Palladium(II)-acetat, Tetrakis-(triphenylphosphin)-palladium(0), Bis-(triphenylphosphin)-palladium(II)-chlorid, Bis-(acetonitril)-palladium(II)-chlorid, [1,1'-Bis(diphenylphosphino)ferrocen]dichlorpalladium(II) und entsprechender Dichlormethan-Komplex, gegebenenfalls in Verbindung mit zusätzlichen Phosphanliganden wie beispielsweise (2-Biphenyl)di-tert.-butylphosphin, Dicyclohexyl[2',4',6'-tris(1-methylethyl)biphenyl-2-yl]phosphan (XPHOS), Bis(2-phenylphosphinophenyl)ether (DPEphos) or 4,5-Bis(diphenylphosphino)-9,9-dimethylxanthen (Xantphos) [vgl. z.B. Hassan J. et al., Chem. Rev. 102, 1359-1469 (2002)] geeignet. Als Kupfer-Katalysatoren eignen sich beispielsweise Kupferbronze, Kupfer-(I)-oxid, Kupfer-(I)-iodid oder Kupfer-(I)-bromid.

Die Umsetzung (II)+ (III) → (I-A) erfolgt in Gegenwart einer geeigneten Base. Geeignete Basen für diese Umsetzung sind die üblichen anorganischen oder organischen Basen. Hierzu gehören bevorzugt Alkalihydroxide wie beispielsweise Lithium-, Natrium- oder Kaliumhydroxid, Alkalioder Erdalkalicarbonate wie Lithium-, Natrium-, Kalium-, Calcium- oder Cäsiumcarbonat, AlkaliAlkoholate wie Natrium- oder Kaliummethanolat, Natrium- oder Kaliumethanolat oder Natrium- oder Kalium-tert.-butylat, Alkalihydride wie Natrium- oder Kaliumhydrid, Amide wie Natriumamid, Lithium-, Natrium- oder Kalium-bis(trimethylsilyl)amid oder Lithiumdiiso-propylamin, oder organische Amine wie Trimethylamin, *N*-Methylmorpholin, N-Methylpiperidin, *N*,*N*-Diisopropylethylamin, Pyridin, 1,5-Diazabicyclo[4.3.0]non-5-en (DBN), 1,8-Diazabicyclo[5.4.0]undec-7-en (DBU) oder 1,4-Diazabicyclo[2.2.2]octan (DABCO^{®}). Bevorzugt wird Natriumhydrid oder Cäsiumcarbonat verwendet.

Die Reaktion (II)+ (III) → (I-A) wird im Allgemeinen in einem Temperaturbereich von 0°C bis +200°C, bevorzugt bei +10°C bis +150°C durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Ist der Rest R^{3A} ungesättigt, kann dieser anschließend vollständig oder teilweise gesättigt werden. Die Reduktion erfolgt mit Wasserstoff in Verbindung mit Übergangsmetallkatalysatoren wie beispielsweise Palladium (10% auf Aktivkohle), Raney-Nickel oder Palladiumhydroxid. Die Reduktion erfolgt im Allgemeinen in einem Temperaturbereich von +20°C bis +50°C. Die Umsetzung kann bei normalem oder erhöhtem Druck durchgeführt werden (z.B. im Bereich von 0.5 bis 5 bar). Im Allgemeinen arbeitet man bei Normaldruck.

Inerte Lösungsmittel für den Verfahrensschritt (IV) + (V) → (I-B) sind beispielsweise Alkohole wie Methanol, Ethanol, n-Propanol, Isopropanol, n-Butanol oder tert.-Butanol, Ether wie Diethylether, Dioxan, Dimethoxyethan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N,N*'-Dimethylpropylenharnstoff (DMPU), *N*-Methylpyrrolidon (NMP), Pyridin oder Acetonitril. Ebenso ist es möglich, Gemische der genannten Lösungsmittel einzusetzen. Bevorzugt ist Methanol oder Ethanol.

Die Reaktion (IV) + (V) → (I-B) erfolgt im Allgemeinen in einem Temperaturbereich von +50°C bis +120°C, bevorzugt von +50°C bis +100°C, gegebenenfalls in einer Mikrowelle. Die Umsetzung kann bei normalem oder erhöhtem Druck durchgeführt werden (z.B. im Bereich von 0.5 bis 5 bar). Im Allgemeinen arbeitet man bei Normaldruck.

Die Umsetzung (I-B) → (VI) kann in einem unter den Reaktionsbedingungen inerten Lösungsmittel oder ohne Lösungsmittel erfolgen. Bevorzugtes Lösungsmittel ist Sulfolan.

Die Reaktion (I-B) → (VI) erfolgt im Allgemeinen in einem Temperaturbereich von +70°C bis +150°C, bevorzugt von +80°C bis +130°C, gegebenenfalls in einer Mikrowelle. Die Umsetzung kann bei normalem oder erhöhtem Druck durchgeführt werden (z.B. im Bereich von 0.5 bis 5 bar). Im Allgemeinen arbeitet man bei Normaldruck.

Insbesondere bevorzugt erfolgt die Umsetzung (I-B) → (VI) ohne Lösungsmittel in einem Temperaturbereich von 0°C bis +50°C bei Normaldruck.

Der Verfahrensschritt (VI) → (I-C) erfolgt in einem unter den Reaktionsbedingungen inerten Lösungsmittel. Geeignete Lösungsmittel sind beispielsweise Ether wie Diethylether, Dioxan, Tetrahydrofuran, Glykoldimethylether oder Diethylenglykoldimethylether, oder anderen Lösungsmitten wie Dimethylformamid (DMF), Dimethylsulfoxid (DMSO), *N,N*'-Dimethylpropylenharnstoff (DMPU), *N*-Methylpyrrolidon (NMP), Pyridin, Acetonitril oder auch Wasser. Ebenso ist es möglich, Gemische der genannten Lösungsmittel einzusetzen. Bevorzugt ist Acetonitril.

Die Reaktion (VI) → (I-C) erfolgt im Allgemeinen in einem Temperaturbereich von +20°C bis +100°C, bevorzugt von +40°C bis +70°C, gegebenenfalls in einer Mikrowelle. Die Umsetzung kann bei normalem oder erhöhtem Druck durchgeführt werden (z.B. im Bereich von 0.5 bis 5 bar). Im Allgemeinen arbeitet man bei Normaldruck.

Bevorzugt erfolgen die Umsetzungen (I-B) → (VI) → (I-C) ohne Isolierung der Zwischenstufe (VI)

Das beschriebene Herstellverfahren kann durch die folgenden Syntheseschemata (Schema 1, 2 und 3) beispielhaft verdeutlicht werden:

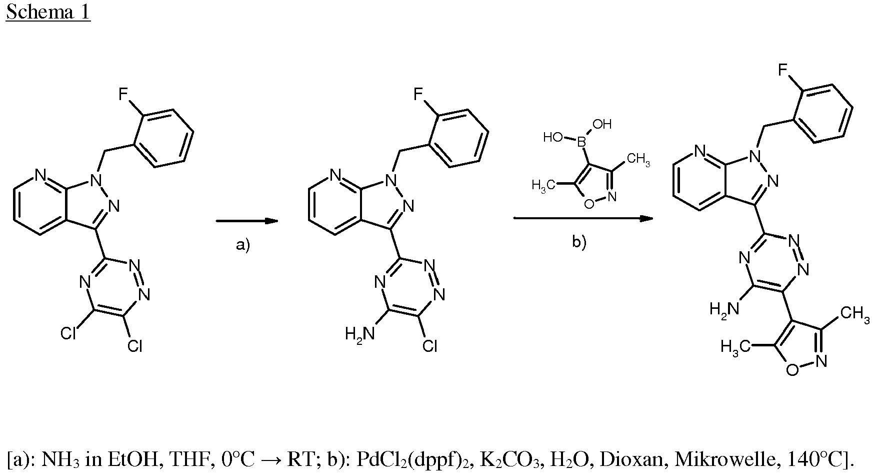

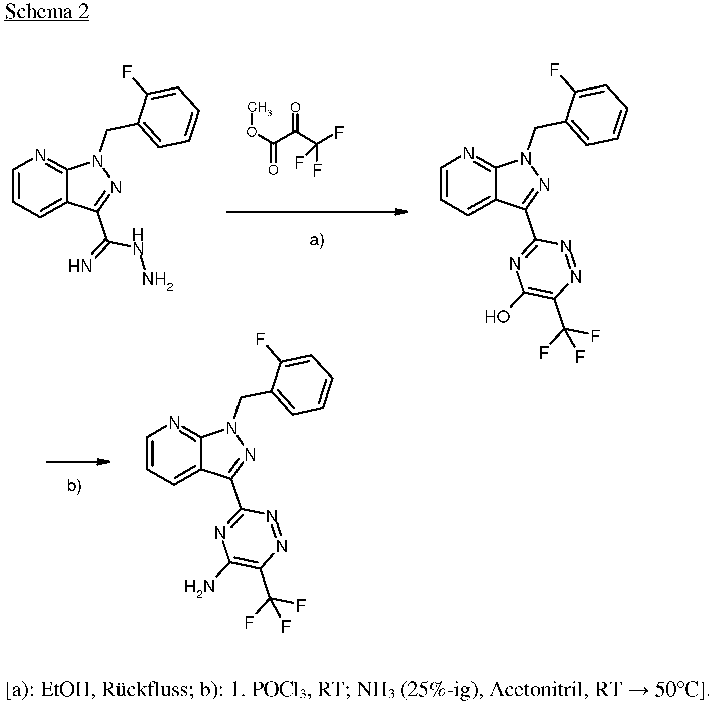

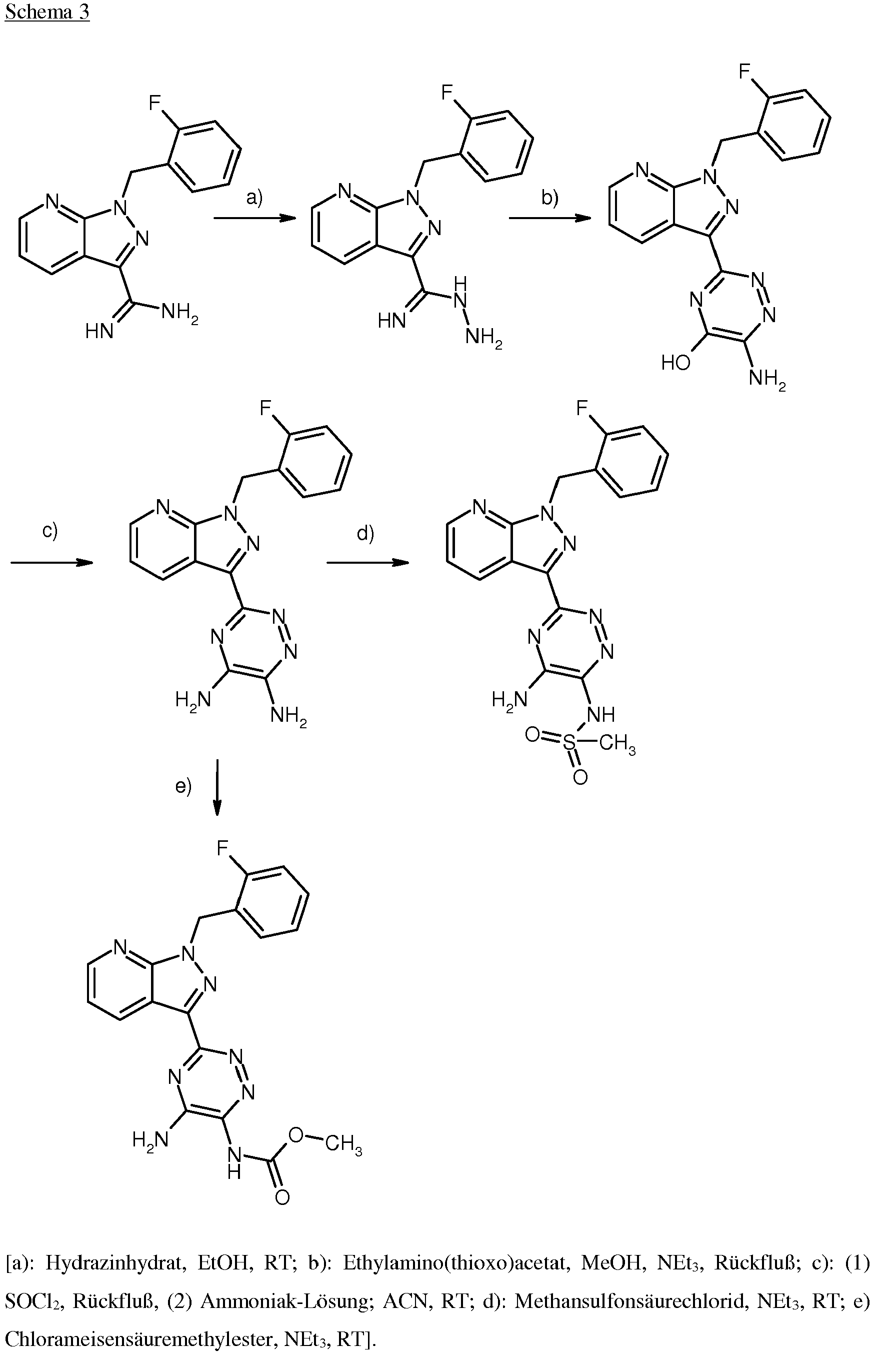

Die Verbindungen der Formel (III) und (V) sind kommerziell erhältlich, literaturbekannt oder können in Analogie zu literaturbekannten Verfahen hergestellt werden.

Weitere erfindungsgemäße Verbindungen können gegebenenfalls auch hergestellt werden durch Umwandlungen von funktionellen Gruppen einzelner Substituenten, insbesondere den unter L und R³ aufgeführten, ausgehend von den nach obigen Verfahren erhaltenen Verbindungen der Formel (I). Diese Umwandlungen werden nach üblichen, dem Fachmann bekannten Methoden durchgeführt und umfassen beispielsweise Reaktionen wie nukleophile und elektrophile Substitutionen, Oxidationen, Reduktionen, Hydrierungen, Übergangsmetall-katalysierte Kupplungsreaktionen, Eliminierungen, Alkylierung, Aminierung, Veresterung, Esterspaltung, Veretherung, Etherspaltung, Bildung von Carbonamide, sowie Einführung und Entfernung temporärer Schutzgruppen.

Die Verbindungen der Formel (II), können hergestellt werden, indem man eine Verbindung der Formel (VI) 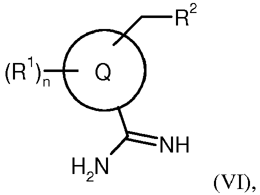 in welcher n, Q, R¹ und R² jeweils die zuvor genannten Bedeutungen haben,
in einem inerten Lösungsmittel in Gegenwart einer geeigneten Base mit Hydrazinhydrat zu einer Verbindung der Formel (IV) 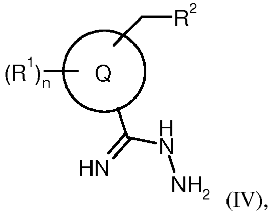 in welcher n, Q, R¹ und R² jeweils die zuvor genannten Bedeutungen haben,
umsetzt, diese anschliessend in einem inerten Lösungsmittel mit Ethyloxoacetat in eine Verbindung der Formel (VII) 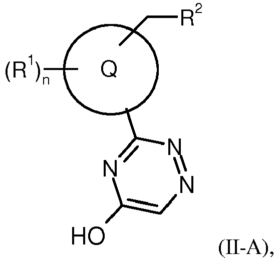 in welcher n, Q, R¹ und R² jeweils die oben genannten Bedeutungen haben,
überführt und diese mit Thionylchlorid zu einer Verbindung der Formel (II) umsetzt.

Das nachfolgende Syntheseschema (Schema 4) verdeutlicht das zuvor beschreibene Verfahren:

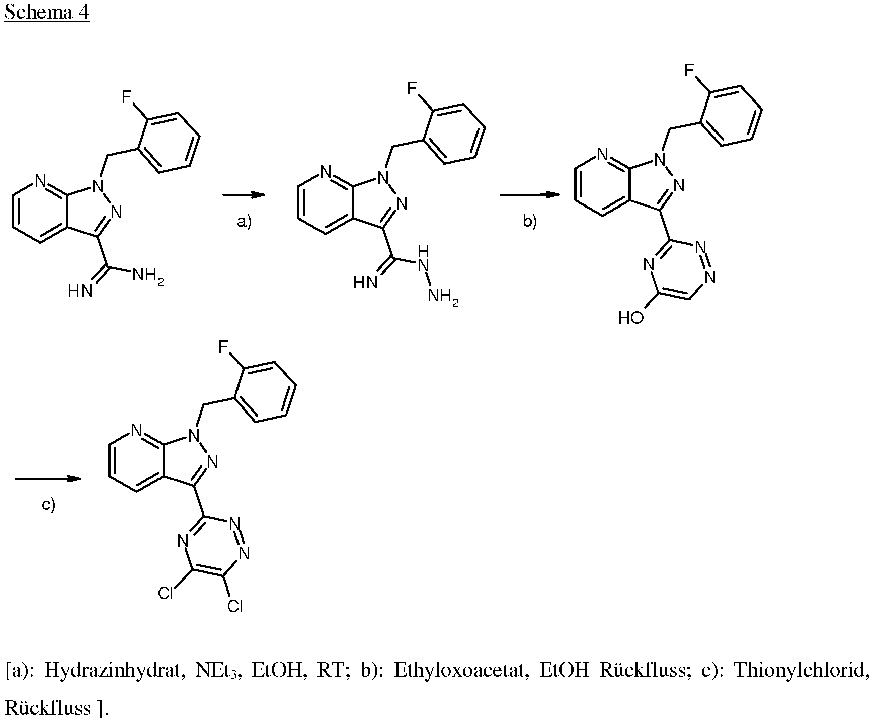

Die Verbindungen der Formel (VI) sind literaturbekannt (siehe z.B. WO 2010/065275, WO 2011/ 115804 und WO 2011/149921) oder können in Analogie zu literaturbekannten Verfahen hergestellt werden.

Die erfindungsgemäßen Verbindungen sind potente Stimulatoren der löslichen Guanylatcyclase, besitzen wertvolle pharmakologische Eigenschaften, und weisen ein verbessertes therapeutisches Profil auf, wie beispielsweise hinsichtlich ihrer *in-vivo* Eigenschaften und/oder ihres pharmakokinetischen Verhaltens. Sie eignen sich daher zur Behandlung und/ oder Prophylaxe von Erkrankungen bei Menschen und Tieren.

Die erfindungsgemäßen Verbindungen bewirken eine Gefäßrelaxation und eine Hemmung der Thrombozytenaggregation und führen zu einer Blutdrucksenkung sowie zu einer Steigerung des koronaren Blutflusses. Diese Wirkungen sind über eine direkte Stimulation der löslichen Guanylatcyclase und einen intrazellulären cGMP-Anstieg vermittelt. Außerdem verstärken die erfindungsgemäßen Verbindungen die Wirkung von Substanzen, die den cGMP-Spiegel steigern, wie beispielsweise EDRF (endothelium-derived relaxing factor), NO-Donatoren, Protoporphyrin IX, Arachidonsäure oder Phenylhydrazin-Derivate.

Die erfindungsgemäßen Verbindungen eignen sich zur Behandlung und/oder Prophylaxe von kardiovaskulären, pulmonalen, thromboembolischen und fibrotischen Erkrankungen.

Die erfindungsgemäßen Verbindungen können daher in Arzneimitteln zur Behandlung und/oder Prophylaxe von kardiovaskulären Erkrankungen wie beispielsweise Bluthochdruck, akute und chronische Herzinsuffizienz, koronare Herzerkrankung, stabile und instabile Angina pectoris, periphere und kardiale Gefäßerkrankungen, Arrhythmien, Rhythmusstörungen der Vorhöfe und der Kammern sowie Überleitungsstörungen wie beispielsweise atrio-ventrikuläre Blockaden Grad I-III (AB-Block I-III), supraventrikuläre Tachyarrhythmie, Vorhofflinmlern, Vorhoffflattern, Kammerflimmern, Kammerflattem, ventrikuläre Tachyarrhytmie, Torsade de pointes-Tachykardie, Extrasystolen des Vorhoffs und des Ventrikels, AV-junktionale Extrasystolen, Sick-Sinus Syndrom, Synkopen, AV-Knoten-Reentrytachykardie, Wolff-Parkinson-White-Syndrom, von akutem Koronarsyndrom (ACS), autoimmune Herzerkrankungen (Perikarditis, Endokarditis, Valvolitis, Aortitis, Kardiomyopathien), Schock wie kardiogenem Schock, septischem Schock und anaphylaktischem Schock, Aneurysmen, Boxerkardiomyopathie (premature ventricular contraction (PVC)), zur Behandlung und/oder Prophylaxe von thromboembolischen Erkrankungen und Ischämien wie myokardiale Ischämie, Myokardinfarkt, Hirnschlag, Herzhypertrophie, transistorischen und ischämischen Attacken, Präeklampsie, entzündliche kardiovaskuläre Erkrankungen., Spasmen der Koronararterien und peripherer Arterien, Ödembildung wie beispielsweise pulmonales Ödem, Hirnödem, renales Ödem oder Herzinsuffizienz-bedingtes Ödem, peripheren Durchblutungsstörungen, Reperfusionsschäden, arterielle und venöse Thrombosen, Mikroalbuminurie, Herzmuskelschwäche, endotheliale Dysfunktion, zur Verhinderung von Restenosen wie nach Thrombolysetherapien, percutan-transluminalen Angioplastien (PTA), transluminalen Koronarangioplastien (PTCA), Herztransplantationen und Bypass-Operationen, sowie mikro- und makrovaskuläre Schädigungen (Vasculitis), erhöhte Spiegel von Fibrinogen und von LDL geringer Dichte sowie erhöhte Konzentrationen von Plasminogenaktivator-Inhibitor 1 (PAI-1), sowie zur Behandlung und/oder Prophylaxe von erektiler Dysfunktion und weiblicher sexueller Dysfunktion eingesetzt werden.

Im Sinne der vorliegenden Erfindung umfasst der Begriff Herzinsuffizienz auch spezifischere oder verwandte Krankheitsformen wie akut dekompensierte Herzinsuffizienz, Rechtsherzinsuffizienz, Linksherzinsuffizienz, Globalinsuffizienz, ischämische Kardiomyopathie, dilatative Kardiomyopathie, hypertrophe Kardiomyopathie, idiopathische Kardiomyopathie, angeborene Herzfehler, Herzklappenfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen, diastolische Herzinsuffizienz sowie systolische Herzinsuffizienz.

Darüber hinaus können die erfindungsgemäßen Verbindungen auch zur Behandlung und/oder Prophylaxe von Arteriosklerose, Lipidstoffwechselstörungen, Hypolipoproteinämien, Dyslipidämien, Hypertriglyceridämien, Hyperlipidämien, Hypercholesterolämien, Abetelipoproteinämie, Sitosterolämie, Xanthomatose, Tangier Krankheit, Fettsucht (Adipositas), Fettleibigkeit (Obesitas) und von kombinierten Hyperlipidämien sowie des Metabolischen Syndroms eingesetzt werden.

Außerdem können die erfindungsgemäßen Verbindungen zur Behandlung und/oder Prophylaxe von primärem und sekundärem Raynaud-Phänomen, von Mikrozirkulationsstörungen, Claudicatio, peripheren und autonomen Neuropathien, diabetischen Mikroangiopathien, diabetischer Retinopathie, diabetischen Geschwüren an den Extremitäten, Gangren, CREST-Syndrom, Erythematose, Onychomykose, rheumatischen Erkrankungen sowie zur Förderung der Wundheilung verwendet werden.

Weiterhin eignen sich die erfindungsgemäßen Verbindungen zur Behandlung urologischer Erkrankungen wie beispielsweise benignes Prostata-Syndrom (BPS), benigne Prostata-Hyperplasie (BPH), benigne Prostata Vergrösserung (BPE), Blasenentleerungsstörung (BOO), untere Harnwegssyndrome (LUTS, einschließlich Felines Urologisches Syndrom (FUS)), Erkrankungen des Urogenital-Systems einschliesslich neurogene überaktive Blase (OAB) und (IC), Inkontinenz (UI) wie beispielsweise Misch-, Drang-, Stress-, oder Überlauf-Inkontinenz (MUI, UUI, SUI, OUI), Beckenschmerzen, benigne und maligne Erkrankungen der Organe des männlichen und weiblichen Urogenital-Systems.

Weiterhin eignen sich die erfindungsgemäßen Verbindungen zur Behandlung und/oder Prophylaxe von Nierenerkrankungen, insbesondere von aktuer und chronischer Niereninsuffizienz, sowie von akutem und chronischem Nierenversagen. Im Sinne der vorliegenden Erfindung umfasst der Begriff Niereninsuffizienz sowohl akute als auch chronische Erscheinungsformen der Niereninsuffizienz, wie auch zugrundeliegende oder verwandte Nierenerkrankungen wie renale Hypoperfusion, intradialytische Hypotonie, obstruktive Uropathie, Glomerulopathien, Glomerulonephritis, akute Glomerulonephritis, Glomerulosklerose, tubulointerstitielle Erkrankungen, nephropathische Erkrankungen wie primäre und angeborene Nierenerkrankung, Nierenentzündung, immunologische Nierenerkrankungen wie Nierentransplantatabstoßung, Inmunkomplex-induzierte Nierenerkrankungen, durch toxische Substanzen induzierte Nephropathie, Kontrastmittelinduzierte Nephropathie, diabetische und nicht-diabetische Nephropathie, Pyelonephritis, Nierenzysten, Nephrosklerose, hypertensive Nephrosklerose und nephrotisches Syndrom, welche diagnostisch beispielsweise durch abnorm verminderte Kreatinin- und/oder Wasser-Ausscheidung, abnorm erhöhte Blutkonzentrationen von Harnstoff, Stickstoff, Kalium und/oder Kreatinin, veränderte Aktivität von Nierenenzymen wie z.B. Glutamylsynthetase, veränderte Urinosmolarität oder Urinmenge, erhöhte Mikroalbuminurie, Makroalbuminurie, Läsionen an Glomerula und Arteriolen, tubuläre Dilatation, Hyperphosphatämie und/oder die Notwendigkeit zur Dialyse charakterisiert werden können. Die vorliegende Erfindung umfasst auch die Verwendung der erfindungsgemäßen Verbindungen zur Behandlung und/oder Prophylaxe von Folgeerscheinungen einer Niereninsuffizienz, wie beispielsweise Lungenödem, Herzinsuffizienz, Urämie, Anämie, Elektrolytstörungen (z.B. Hyperkalzämie, Hyponaträmie) und Störungen im Knochen- und Kohlenhydrat-Metabolismus.

Weiterhin eignen sich die erfindungsgemäßen Verbindungen auch zur Behandlung und/oder Prophylaxe von asthmatischen Erkrankungen., pulmonaler arterieller Hypertonie (PAH) und anderen Formen der pulmonalen Hypertonie (PH), umfassend mit Linksherzerkrankung, HIV, Sichelzellanämie, Thromboembolien (CTEPH), Sarkoidose, COPD oder Lungenfibrose assoziierte pulmonale Hypertonie, der chronisch-obstruktive Lungenerkrankung (COPD), des akuten Atemwegssyndrom (ARDS), der akuten Lungenschädigung (ALI), der alpha-1-Antitrypsin-Defizienz (AATD), der Lungenfibrose, des Lungenemphysem (z.B. durch Zigarettenrauch induziertes Lungenemphysem) und der zystischen Fibrose (CF).

Die in der vorliegenden Erfindung beschriebenen Verbindungen stellen auch Wirkstoffe zur Bekämpfung von Krankheiten im Zentralnervensystem dar, die durch Störungen des NO/cGMP-Systems gekennzeichnet sind. Insbesondere sind sie geeignet zur Verbesserung der Wahrnehmung, Konzentrationsleistung, Lernleistung oder Gedächtnisleistung nach kognitiven Störungen, wie sie insbesondere bei Situationen/Krankheiten/Syndromen auftreten wie "Mild cognitive impairment", altersassoziierten Lern- und Gedächtnisstörungen, altersassoziierten Gedächtnisverlusten, vaskulärer Demenz, Schädel-Hirn-Trauma, Schlaganfall, Demenz, die nach Schlaganfällen auftritt ("post stroke dementia"), post-traumatischem Schädel-Hirn-Trauma, allgemeinen Konzentrationsstörungen, Konzentrationsstörungen bei Kindern mit Lern- und Gedächtnisproblemen, Alzheimer'scher Krankheit, Demenz mit Lewy-Körperchen, Demenz mit Degeneration der Frontallappen einschliesslich des Pick's-Syndroms, Parkinson'scher Krankheit, progressiver nuclear palsy, Demenz mit corticobasaler Degeneration, Amyolateralsklerose (ALS), Huntington'scher Krankheit, Demyelinisation, Multipler Sklerose, Thalamischer Degeneration, Creutzfeld-Jacob-Demenz, HIV-Demenz, Schizophrenie mit Demenz oder Korsakoff-Psychose. Sie eignen sich auch zur Behandlung und/oder Prophylaxe von Erkrankungen des Zentralnervensystems wie Angst-, Spannungs- und Depressionszuständen, zentral-nervös bedingten Sexualdysfunktionen und Schlafstörungen sowie zur Regulierung krankhafter Störungen der Nahrungs-, Genuss- und Suchtmittelaufnahme.

Weiterhin eignen sich die erfindungsgemäßen Verbindungen auch zur Regulation der cerebralen Durchblutung und stellen wirkungsvolle Mittel zur Bekämpfung von Migräne dar. Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfindungsgemäßen Verbindungen zur Bekämpfung von Schmerzzuständen und Tinnitus eingesetzt werden.

Zudem besitzen die erfindungsgemäßen Verbindungen antiinflammatorische Wirkung und können daher als entzündungshenmlende Mittel zur Behandlung und/oder Prophylaxe von Sepsis (SIRS), multiplem Organversagen (MODS, MOF), entzündlichen Erkrankungen der Niere, chronischen Darmentzündungen (IBD, Crohn's Disease, UC), Pankreatitis, Peritonitis, rheumatoiden Erkrankungen, entzündlichen Hauterkrankungen sowie entzündlichen Augenerkrankungen eingesetzt werden.

Des weiteren können die erfindungsgemäßen Verbindungen ebenfalls zur Behandlung und/ oder Prophylaxe von Autoimmunerkrankungen eingesetzt werden.

Weiterhin sind die erfindungsgemäßen Verbindungen zur Behandlung und/oder Prophylaxe fibrotischer Erkrankungen der inneren Organe, wie beispielsweise der Lunge, des Herzens, der Niere, des Knochenmarks und insbesondere der Leber, sowie dermatologischer Fibrosen und fibrotischer Erkrankungen des Auges, geeignet. Im Sinne der vorliegenden Erfindungen umfasst der Begriff fibrotischer Erkrankungen insbesondere die folgenden Begriffe Leberfibrose, Leberzirrhose, Lungenfibrose, Endomyocardfibrose, Nephropathie, Glomerulonephritis, interstitielle Nierenfibrose, fibrotische Schäden in Folge von Diabetes, Knochenmarksfibrose und ähnliche fibrotische Erkrankungen, Sklerodermie, Morphaea, Keloide, hypertrophe Narbenbildung (auch nach chirurgischen Eingriffen), Naevi, diabetische Retinopathie, proliferative Vitroretinopathie und Erkrankungen des Bindegewebes (z.B. Sarkoidose).

Weiterhin eignen sich die erfindungsgemäßen Verbindungen zur Bekämpfung postoperativer Narbenbildung, z.B. in Folge von Glaukom-Operationen.

Die erfindungsgemäßen Verbindungen können ebenfalls kosmetisch bei alternder und verhornender Haut eingesetzt werden.

Außerdem sind die erfindungsgemäßen Verbindungen zur Behandlung und/ oder Prophylaxe von Hepatitis, Neoplasma, Osteoporose, Glaukom und Gastroparese geeignet.

Weiterer Gegenstand der vorliegenden Erfindung sind die erfindungsgemäßen Verbindungen zur Behandlung und/oder Prophylaxe von Erkrankungen, insbesondere der zuvor genannten Erkrankungen.

Weiterer Gegenstand der vorliegenden Erfindung sind die erfindungsgemäßen Verbindungen zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Weiterer Gegenstand der vorliegenden Erfindung sind die erfindungsgemäßen Verbindungen zur Verwendung in einem Verfahren zur Behandlung und/ oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Weiterer Gegenstand der vorliegenden Erfindung ist die Verwendung der erfindungsgemäßen Verbindungen zur Herstellung eines Arzneimittels zur Behandlung und/oder Prophylaxe von Erkranzungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Weiterer Offenbarungsgegestand ist ein Verfahren zur Behandlung und/oder Prophylaxe von Erkrankungen, insbesondere der zuvor genannten Erkrankungen., unter Verwendung einer wirksamen Menge von mindestens einer der erfindungsgemäßen Verbindungen.

Weiterer Offenbarungsgegenstand ist ein Verfahren zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose, unter Verwendung einer wirksamen Menge von mindestens einer der erfindungsgemäßen Verbindungen.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prophylaxe der zuvor genannten Erkrank-ungen. Als geeignete Kombinationswirkstoffe seien beispielhaft und vorzugsweise genannt:
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- Verbindungen, die den Abbau von cyclischem Guanosinmonophosphat (cGMP) inhibieren, wie beispielsweise Inhibitoren der Phosphodiesterasen (PDE) 1, 2 und/oder 5, insbesondere PDE 5-Inhibitoren wie Sildenafil, Vardenafil und Tadalafil;
- antithrombotisch wirkende Mittel, beispielhaft und vorzugsweise aus der Gruppe der Thrombozytenaggregationshemmer, der Antikoagulantien oder der profibrinolytischen Substanzen;
- den Blutdruck senkende Wirkstoffe, beispielhaft und vorzugsweise aus der Gruppe der Calcium-Antagonisten, Angiotensin AII-Antagonisten, ACE-Henmler, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika; und/oder
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten.

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Dabigatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban (BAY 59-7939), DU-176b, Apixaban, Otamixaban, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitasin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embursatan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Schleifendiuretikum, wie beispielsweise Furosemid, Torasemid, Bumetanid und Piretanid, mit kaliumsparenden Diuretika wie beispielsweise Amilorid und Triamteren, mit Aldosteronantagonisten, wie beispielsweise Spironolacton, Kaliumcanrenoat und Eplerenon sowie Thiaziddiuretika, wie beispielsweise Hydrochlorothiazid, Chlorthalidon, Xipamid, und Indapamid, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-Inhibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Dalcetrapib, BAY 60-5521, Anacetrapib oder CETP-vaccine (CETi-1), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-Inhibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.001 bis 2 mg/kg, vorzugsweise etwa 0.001 bis 1 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen und Akronyme:

- aq.: wässrige Lösung
- ber.: Berechnet
- DCI: direkte chemische Ionisation (bei MS)
- DMF: Dimethylformamid
- DMSO: Dimethylsulfoxid
- d. Th.: der Theorie (bei Ausbeute)
- eq.: Äquivalent(e)
- ESI: Elektrospray-Ionisation (bei MS)
- Et: Ethyl
- gef.: Gefunden
- h: Stunde(n)
- HPLC: Hochdruck-, Hochleistungsflüssigchromatographie
- HRMS: hochaufgelöste Massenspektrometrie
- konz.: konzentriert
- LC/MS: Flüssigchromatographie-gekoppelte Massenspektrometrie
- LiHMDS: Lithiumhexamethyldisilazid
- Me: Methyl
- min: Minute(n)
- MS: Massenspektrometrie
- NMR: Kernresonanzspektrometrie
- Pd/C: Palladium auf Aktivkohle (10%-fig)
- Ph: Phenyl
- RT: Raumtemperatur
- Rₜ: Retentionszeit (bei HPLC)
- t-Bu: tert.-Butyl
- TFA: Trifluoressigsäure
- THF: Tetrahydrofuran
- UV: Ultraviolett-Spektrometrie
- v/v: Volumen zu Volumen-Verhältnis (einer Lösung)
- XPHOS: Dicyclohexyl-(2',4',6'-trüsopropylbiphenyl-2-yl)-phosphin

### LC/MS-Methoden:

### Methode 1:

Instrument: Waters ACQUITY SQD UPLC System; Säule: Waters Acquity UPLC HSS T3 1.8 µ 50 x 1 mm; Eluent A: 11 Wasser + 0.25 ml 99%ige Ameisensäure , Eluent B: 11 Acetonitril + 0.25 ml 99%ige Ameisensäure; Gradient: 0.0 min 90% A → 1.2 min 5% A → 2.0 min 5% A Ofen: 50°C; Fluss: 0.40 ml/min; UV-Detektion: 208 - 400 nm.

### Methode 2:

Instrument: Waters ACQUITY SQD UPLC System; Säule: Waters Acquity UPLC HSS T3 1.8 µ 30 x 2 mm; Eluent A: 11 Wasser + 0.25 ml 99%ige Ameisensäure , Eluent B: 11 Acetonitril + 0.25 ml 99%ige Ameisensäure; Gradient: 0.0 min 90% A → 1.2 min 5% A → 2.0 min 5% A Ofen: 50°C; Fluss: 0.60 ml/min; UV-Detektion: 208 - 400 nm.

### Methode 3:

Instrument: Micromass Quattro Premier mit Waters UPLC Acquity; Säule: Thermo Hypersil GOLD 1.9 µ 50 x 1 mm; Eluent A: 11 Wasser + 0.5 ml 50%ige Ameisensäure, Eluent B: 11 Acetonitril + 0.5 ml 50%ige Ameisensäure; Gradient: 0.0 min 90% A → 0.1 min 90% A → 1.5 min 10% A → 2.2 min 10% A Ofen: 50°C; Fluss: 0.33 ml/min; UV-Detektion: 210 nm.

### Methode 4:

Instrument: Waters ACQUITY SQD UPLC System; Säule: Waters Acquity UPLC HSS T3 1.8 µ 50 x 1 mm; Eluent A: 11 Wasser + 0.25 ml 99%ige Ameisensäure , Eluent B: 11 Acetonitril + 0.25 ml 99%ige Ameisensäure; Gradient: 0.0 min 95% A → 6.0 min 5% A → 7.5 min 5% A Ofen: 50°C; Fluss: 0.35 ml/min; UV-Detektion: 210 - 400 nm.

### Methode 5:

Instrument: Micromass Quattro Premier mit Waters UPLC Acquity; Säule: Thermo Hypersil GOLD 1.9 µ 50 x 1 mm; Eluent A: 11 Wasser + 0.5 ml 50%ige Ameisensäure, Eluent B: 11 Acetonitril + 0.5 ml 50%ige Ameisensäure; Gradient: 0.0 min 97% A → 0.5 min 97% A → 3.2 min 5% A → 4.0 min 5% A Ofen: 50°C; Fluss: 0.3 ml/min; UV-Detektion: 210 nm.

### Ausgangsverbindungen und Intermediate:

### Beispiel 1A

### 1-(2-Fluorbenzyl)-1H-pyrazolo[3,4-b]pyridin-3-carboximidamid-Hydrochlorid

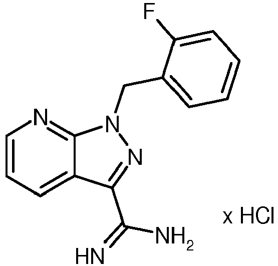

Die Synthese dieser Verbindung ist beschrieben in WO 03/095451, Beispiel 6A.

### Beispiel 2A

### 1-(2-Fluorbenzyl)-1H-pyrazolo[3,4-b]pyridin-3-carboximidohydrazid

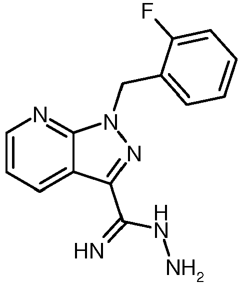

50.000 g (163.535 mmol) 1-(2-Fluorbenzyl)-1H-pyrazolo[3,4-b]pyridin-3-carboximidamid-Hydrochlorid wurden in 700 ml Ethanol gelöst und bei 0°C mit 66.192 g (654.141 nmlol) Trimethylamin sowie 10.233 g (163.535 mmol) Hydrazinhydrat (80%-ige Lösung in Wasser) versetzt. Das Gemische wurde über Nacht bei RT gerührt und anschließend am Rotationsverdampfer eingeengt. Der Rückstand wurde in Essigsäureethylester aufgenommen und die Lösung dreimal mit gesättigter wässriger Natriumchlorid-Lösung gewaschen. Die organische Phase wurde über Natriumsulfat getrocknet und am Rotationsverdampfer eingeengt. Der Rückstand wurde mit Diethylether verrührt, abgesaugt und im Hochvakuum getrocknet. Es wurden 46.49 g (46 % d. Th., 68%-ige Reinheit) der Titelverbindung erhalten.
LC-MS (Methode 5): Rₜ = 0.64 min; MS (ESIpos): m/z = 285 (M+H)⁺

### Beispiel 3A

### 3-[1-(2-Fluorbenzyl)-1H-pyrazolo[3,4-b]pyridin-3-yl]-1,2,4-triazin-5-ol

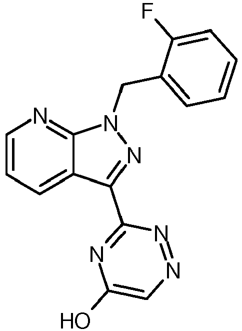

Es wurden 22.000 g (Reinheit 68%, ca. 52.621 mmol) 1-(2-Fluorbenzyl)-1H-pyrazolo[3,4-b]pyridin-3-carboximidohydrazid in 220 ml Ethanol vorgelegt, mit 18.265 (89.455 mmol) Ethyloxoacetat (50%-ige Lösung in Toluol) tropfenweise versetzt und über Nacht zum Rückfluss erhitzt. Die entstandene Suspension wurde am Rotationsverdampfer eingeengt und mit Diethylether verrührt. Der Feststoff wurde abgesaugt und im Hochvakuum getrocknet. Weitere Reinigung erfolgte durch Chromatographie an Kieselgel (Eluent: Dichlormethan/Methanol, Gradient 30:1 → 10:1). Es wurden 12.07 g der Zielverbindung erhalten (Reinheit 69%, 49% d. Th.).
LC-MS (Methode 1): Rₜ = 0.80 min; MS (ESIpos): m/z = 323 (M+H)⁺

### Beispiel 4A

### 3-(5,6-Dichlor-1,2,4-triazin-3-yl)-1-(2-fluorbenzyl)-1H-pyrazolo[3,4-b]pyridin

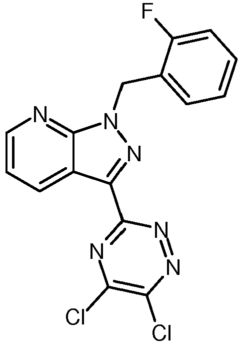

Es wurden 12.000 g (Reinheit 69%, ca. 25.690 mmol) 3-[1-(2-Fluorbenzyl)-1H-pyrazolo[3,4-b]pyridin-3-yl]-1,2,4-triazin-5-ol in 70 ml Thionylchlorid für 6h zum Rückfluss erhitzt. Die Reaktionsmischung wurde am Rotationsverdampfer eingeengt und mit Toluol versetzt, erneut eingeengt und im Hochvakuum getrocknet. Es wurden 13.10 g der Zielverbindung erhalten (Reinheit 38%, 52% d. Th.).
LC-MS (Methode 1): Rₜ = 1.22 min; MS (ESIpos): m/z = 375 (M+H)⁺

### Beispiel 5A

### 6-Chlor-3-[1-(2-fluorbenzyl)-1H-pyrazolo[3,4-b]pyridin-3-yl]-1,2,4-triazin-5-amin

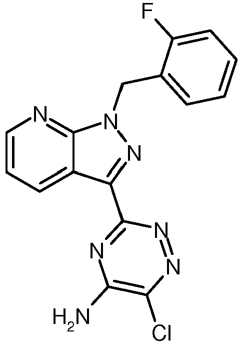

Es wurden 7.000 g (Reinheit 38%, 7.090 mmol) 3-(5,6-Dichlor-1,2,4-triazin-3-yl)-1-(2-fluorbenzyl)-1H-pyrazolo[3,4-b]pyridin in 200 ml absolutem THF vorgelegt. Unter Eiskühlung wurden 4.254 ml (8.508 mmol) 2N Ammoniak-Lösung in Ethanol zugegeben und 1h bei 0°C gerührt. Es wurden erneut unter Eiskühlung 4.254 ml (8.508 mmol) 2N Ammoniak-Lösung in Ethanol zugegeben und 1.5h bei RT gerührt. Es wurden 30 ml (60.000 mmol) 2N Ammoniak-Lösung in Ethanol addiert und 15 min bei RT gerührt. Das Reaktionsgemisch wurde einrotiert, in 100 ml Dichlormethan suspendiert, mit 50 ml (100.00 mmol) 2N Ammoniak-Lösung in Ethanol versetzt und 2h bei RT gerührt. Die Mischung wurde einrotiert und durch Chromatographie an Kieselgel (Eluent: Dichlormethan/Methanol, Gradient 20:1 → 10:1) gereinigt. Die produkthaltigen Fraktionen wurden eingeengt und mit DMSO verrührt. Der Feststoff wurde abgesaugt mit Acetonitril nachgewaschen und im Hochvakuum getrocknet. Der Rückstand wurde mittels präparativer HPLC (Eluent: Acetonitril/Wasser, Gradient 20:80 → 100:0). Es wurden 1.68 g der Zielverbindung erhalten (Reinheit 65%, 43% d. Th.).
LC-MS (Methode 1): Rₜ = 0.87 min; MS (ESIpos): m/z = 356 (M+H)⁺

### Beispiel 6A

### 6-Amino-3-[1-(,2-fluorbenzyl)-1H-pyrazolo[3,4-b]pyridin-3-yl]-1,2,4-triazin-5-ol

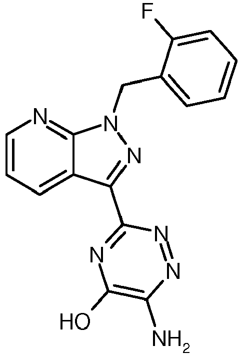

Es wurden 2.000 g (7.035 mmol) 1-(2-Fluorbenzyl)-1H-pyrazolo[3,4-b]pyridin-3-carboximidohydrazid in 50 ml Methanol vorgelegt und mit 0.937 g (7.035 mmol) Ethylamino(thioxo)acetat, sowie 1.424 g (14.070 mmol) Trimethylamin versetzt und 5h zum Rückfluss erhitzt. Die Reaktionsmischung wurde über Nacht stehen gelassen, der Niederschlag abgesaugt, mit wenig Ethanol gewaschen und im Hochvakuum getrocknet. Es wurden 1.892 g der Zielverbindung erhalten (Reinheit 94%, 75% d. Th.).
LC-MS (Methode 1): Rₜ = 0.72 min; MS (ESIpos): m/z = 338 (M+H)⁺

### Beispiel 7A

Ethyl-2-fluor-2-13-[1-(2-fluorbenzyl)-1H-pyrazolo[3,4-b]pyridin-3-yl]-5-hydroxy-1,2,4-triazin-6-yl}propanoat

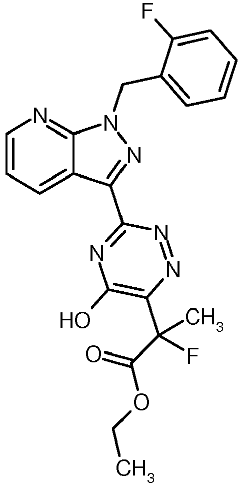

Es wurden 1.000 g (Reinheit 67%, ca. 2.357 mmol) 1-(2-Fluorbenzyl)-1H-pyrazolo[3,4-b]pyridin-3-carboximidohydrazid in 15 ml absolutem Ethanol vorgelegt und mit 1.557 g (7.070 mmol) Diethyl-2-fluor-2-methyl-3-oxobutandioat (beschrieben in J. Med. Chem. 1966 , 9, 149 - 151) versetzt. Das Gemische wurde über Nacht bei RT gerührt und anschließend eingeengt. Der Rückstand wurde mittels präparativer HPLC (Eluent: Methanol/Wasser, Gradient 30:70 → 95:5) gereinigt. Es wurden 230 mg der Zielverbindung erhalten (Reinheit 95%, 21% d. Th.).
LC-MS (Methode 2): Rₜ = 1.01 min; MS (ESIpos): m/z = 441 (M+H)⁺

### Beispiel 8A

2-{5-Amino-3-1-(2-fluorbenzyl)-1H-pyrazolo[3,4-b]pyridin-3-yl]-1,2,4-triazin-6-yl-2-fluorpropanamid

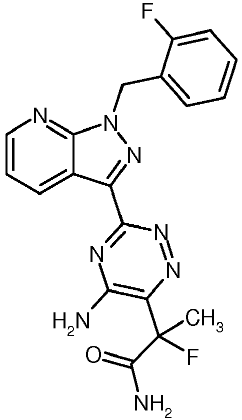

Es wurden 250 mg (0.522 mmol) Ethyl-2-fluor-2-{3-[1-(2-fluorbenzyl)-1H-pyrazolo[3,4-b]pyridin-3-yl]-5-hydroxy-1,2,4-triazin-6-yl}propanoat mit 3 ml Phosphorylchlorid versetzt und über Nacht bei RT gerührt. Das Reaktionsgemisch wurde mit 10 ml trockenem Acetonitril verdünnt und unter Eiskühlung in 5 ml konzentrierte wässrige Ammoniak-Lösung (35%-ig) eingerührt. Es wurde für 2h bei RT und 16h bei 50°C gerührt. Nach dem Abkühlen wurden wurde der Niederschlag abgesaugt und im Vakuum getrocknet. Es wurden 294 mg (Reinheit 95%, quant. Ausbeute) der Zielverbindung erhalten.
LC-MS (Methode 3) Rₜ = 0.96 min; MS (ESIpos): m/z = 411 (M+H)⁺

### Ausführungsbeispiele:

### Beispiel 1

### 3-[1-(2-Fluorbenzyl)-1H-pyrazolo[3,4-b]pyridin-3-yl]-6-(5-fluorpyridin-3-yl)-1,2,4-triazin-5-amin

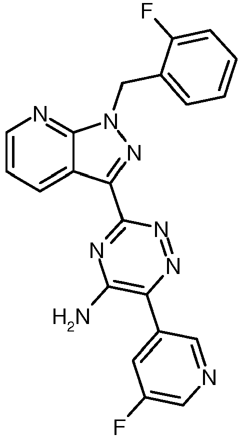

Unter einer Argonatmosphäre wurden 100 mg (Reinheit 65%, 0.183 mmol) 6-Chlor-3-[1-(2-fluorbenzyl)-1H-pyrazolo[3,4-b]pyridin-3-yl]-1,2,4-triazin-5-amin in 4 ml absolutem Dioxan suspendiert. Es wurden 103 mg (0.731 mmol) (5-Fluorpyridin-3-yl)boronsäure und 25 mg (0.183 mmol) Kaliumcarbonat addiert und 10 min unter Rühren Argon durch die Suspension geleitet. Anschließend wurden 3 mg (4.020 µmol) 1,1'-Bis(diphenylphosphino)ferrocenpalladium(II)chlorid zugegeben und erneut für 1 min Argon durch die Mischung geleitet. Das Reaktionsgemisch wurde für 20 min in der Mikrowelle bei 140°C gerührt. Es wurden 5 mg (0.018 mmol) Tricyclohexylphosphin addiert und erneut für 20 Min. in der Mikrowelle bei 140°C gerührt. Nach dem Abkühlen wurde die Mischung über eine Extrelut-Kartusche filtriert und mit einem Gemische aus Dichlormethan/Methanol (v/v = 20:1) nachgewaschen. Das Filtrat wurde eingeengt und der Rückstand mittels präparativer HPLC (Eluent: Acetonitril/Wasser, Gradient 20:80 → 100:0) gereinigt. Es wurden 59 mg der Zielverbindung erhalten (58% d. Th.).
LC-MS (Methode 4): Rₜ = 4.78 min; MS (ESIpos): m/z = 417 (M+H)⁺
¹H-NMR (400 MHz, DMSO-d₆): δ [ppm] = 5.92 (s, 2H), 7.17 (t, 1H), 7.23-7.27 (m, 3H), 7.35-7.41 (m, 1H), 7.51 (dd, 1H), 8.08 (dt, 1H), 8.73-8.78 (m, 3H), 8.96 (dd, 1H).

### Beispiel 2

### 3-[1-(2-Fluorbenzyl)-1H-pyrazolo[3,4-b]pyridin-3-yl]-6-(,2-methylpyridin-3-yl)-1,2,4-triazin-5-amin

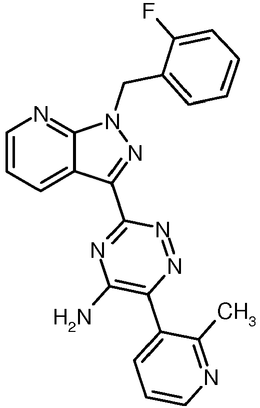

Unter einer Argonatmosphäre wurden 140 mg (Reinheit 65%, 0.256 mmol) 6-Chlor-3-[1-(2-fluorbenzyl)-1H-pyrazolo[3,4-b]pyridin-3-yl]-1,2,4-triazin-5-amin in 5 ml absolutem Dioxan suspendiert. Es wurden 105 mg (0.767 mmol) (2-Methylpyridin-3-yl)boronsäure, 1.023 ml (1.023 mmol) 1N wässrige Kaliumcarbonat-Lösung sowie 14 mg (0.051 mmol) Tricyclohexylphosphin addiert und 10 min unter Rühren Argon durch die Suspension geleitet. Anschließend wurden 28 mg (0.038 mmol) 1,1'-Bis(diphenylphosphino)ferrocenpalladium(II)chlorid zugegeben und das Reaktionsgemisch für 30 min in der Mikrowelle bei 140°C gerührt. Es wurden erneut 19 mg (0.026 mmol) 1,1'-Bis(diphenylphosphino)ferrocenpalladium(II)chlorid zugegeben und das Reaktionsgemisch für 40 min in der Mikrowelle bei 150°C gerührt. Nach dem Abkühlen wurde die Mischung über eine Extrelut-Kartusche filtriert und mit einem Gemische aus Dichlormethan/Methanol (v/v = 20:1) nachgewaschen. Das Filtrat wurde eingeengt und der Rückstand mittels präparativer HPLC (Eluent: Acetonitril/Wasser, Gradient 20:80 → 100:0) gereinigt. Es wurden 26 mg der Zielverbindung erhalten (18% d. Th.).
LC-MS (Methode 4): Rₜ = 4.27 min; MS (ESIpos): m/z = 413 (M+H)⁺
¹H-NMR (400 MHz, DMSO-d₆): δ [ppm] = 2.48 (s, 3H), 5.91 (s, 2H), 7.17 (t, 1H), 7.22-7.27 (m, 2H), 7.35-7.41 (m, 1H), 7.49 (dd, 1H), 7.68 (t, 1H), 8.14 (d, 1H), 8.73 (dd, 1H), 8.77 (d, 1H), 8.95 (dd, 1H).

### Beispiel 3

### 6-(3,5-Dimethyl-1,2-oxazol-4-yl)-3-[1-(2-fluorbenzyl)-1H-pyrazolo[3,4-b]pyridin-3-yl]-1,2,4-triazin-5-amin

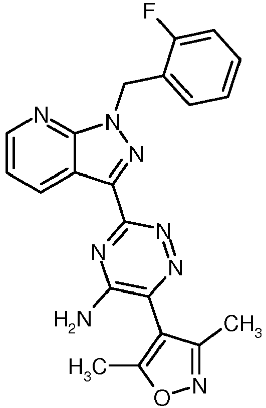

Unter einer Argonatmosphäre wurden 140 mg (Reinheit 65%, 0.256 mmol) 6-Chlor-3-[1-(2-fluorbenzyl)-1H-pyrazolo[3,4-b]pyridin-3-yl]-1,2,4-triazin-5-amin in 5 ml absolutem Dioxan suspendiert. Es wurden 154 mg (Reinheit 70%, 0.767 mmol) (3,5-Dimethyl-1,2-oxazol-4-yl)boronsäure, 1.023 ml (1.023 mmol) 1N wässrige Kaliumcarbonat-Lösung sowie 14 mg (0.051 mmol) Tricyclohexylphosphin addiert und 10 min unter Rühren Argon durch die Suspension geleitet. Anschließend wurden 28 mg (0.038 mmol) 1,1'-Bis(diphenylphosphino)ferrocen-palladium(II)chlorid zugegeben und das Reaktionsgemisch für 30 min in der Mikrowelle bei 140°C gerührt. Es wurden erneut das Reaktionsgemisch für 60 min in der Mikrowelle bei 140°C gerührt. Nach dem Abkühlen wurde die Mischung über eine Extrelut-Kartusche filtriert und mit einem Gemische aus Dichlormethan/Methanol (v/v = 20:1) nachgewaschen. Das Filtrat wurde eingeengt und der Rückstand mittels präparativer HPLC (Eluent: Acetonitril/Wasser, Gradient 20:80 → 100:0) gereinigt. Es wurden 25 mg der Zielverbindung erhalten (Reinheit 95%, 18% d. Th.).
LC-MS (Methode 4): Rₜ = 4.73 min; MS (ESIpos): m/z = 417 (M+H)⁺
¹H-NMR (400 MHz, DMSO-d₆): δ [ppm] = 2.22 (s, 3H), 2.40 (s, 3H), 5.90 (s, 2H), 7.16 (t, 1H), 7.21-7.27 (m, 2H), 7.35-7.41 (m, 1H), 7.48 (dd, 1H), 8.72 (dd, 1H), 8.94 (dd, 1H).

### Beispiel 4

3-[1-(2-Fluorbenzyl)-1H-pyrazolo[3,4-b]pyridin-3-yl]-6-(6-methoxypyridin-3-yl)-1,2,4-triazin-5-amin

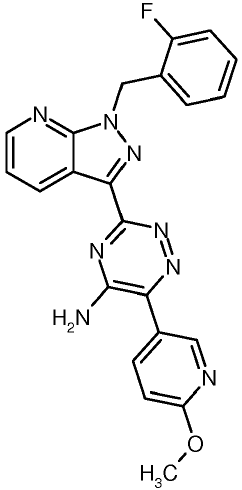

Unter einer Argonatmosphäre wurden 140 mg (Reinheit 65%, 0.256 mmol) 6-Chlor-3-[1-(2-fluorbenzyl)-1H-pyrazolo[3,4-b]pyridin-3-yl]-1,2,4-triazin-5-amin in 5 ml absolutem Dioxan suspendiert. Es wurden 117 mg (0.767 mmol) (6-Methoxypyridin-3-yl)boronsäure, 1.023 ml (1.023 mmol) 1N wässrige Kaliumcarbonat-Lösung sowie 14 mg (0.051 mmol) Tricyclohexylphosphin addiert und 10 min unter Rühren Argon durch die Suspension geleitet. Anschließend wurden 28 mg (0.038 mmol) 1,1'-Bis(diphenylphosphino)ferrocenpalladium(II)chlorid zugegeben und das Reaktionsgemisch für 30 min in der Mikrowelle bei 140°C gerührt. Nach dem Abkühlen wurde die Mischung über eine Extrelut-Kartusche filtriert und mit einem Gemische aus Dichlormethan/Methanol (v/v = 20:1) nachgewaschen. Das Filtrat wurde eingeengt und der Rückstand mittels präparativer HPLC (Eluent: Acetonitril/Wasser, Gradient 20:80 → 100:0) gereinigt. Es wurden 58 mg der Zielverbindung erhalten (Reinheit 85%, 36% d. Th.).
LC-MS (Methode 4): Rₜ = 4.77 min; MS (ESIpos): m/z = 429 (M+H)⁺
¹H-NMR (400 MHz, DMSO-d₆): δ [ppm] = 3.96 (s, 3H), 5.92 (s, 2H), 7.02 (d, 1H), 7.16 (t, 1H), 7.21-7.27 (m, 2H), 7.36-7.40 (m, 1H), 7.51 (dd, 1H), 8.03 (dd, 1H), 8.53 (d, 1H), 8.74 (dd, 1H), 8.95 (dd, 1H).

### Beispiel 5

### 3-[1-(2-Fluorbenzyl)-1H-pyrazolo[3,4-b]pyridin-3-yl]-6-(pyridin-4-yl)-1,2,4-triazin-5-amin

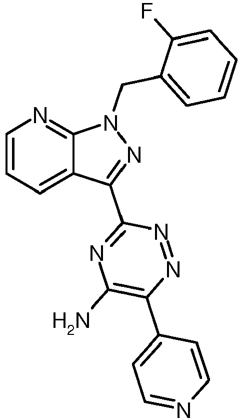

Unter einer Argonatmosphäre wurden 140 mg (Reinheit 65%, 0.256 mmol) 6-Chlor-3-[1-(2-fluorbenzyl)-1H-pyrazolo[3,4-b]pyridin-3-yl]-1,2,4-triazin-5-amin in 5 ml absolutem Dioxan suspendiert. Es wurden 122 mg (0.767 mmol) Pyridin-4-ylboronsäure-Hydrochlorid, 1.023 ml (1.023 mmol) 1N wässrige Kaliumcarbonat-Lösung sowie 14 mg (0.051 mmol) Tricyclohexylphosphin addiert und 10 min unter Rühren Argon durch die Suspension geleitet. Anschließend wurden 28 mg (0.038 mmol) 1,1'-Bis(diphenylphosphino)ferrocen-palladium(II)chlorid zugegeben und das Reaktionsgemisch für 30 min in der Mikrowelle bei 140°C gerührt. Es wurden erneut das Reaktionsgemisch für 60 min in der Mikrowelle bei 140°C gerührt. Nach dem Abkühlen wurde die Mischung über eine Extrelut-Kartusche filtriert und mit einem Gemische aus Dichlormethan/Methanol (v/v = 20:1) nachgewaschen. Das Filtrat wurde eingeengt und der Rückstand mittels präparativer HPLC (Eluent: Acetonitril/Wasser, Gradient 20:80 → 100:0) gereinigt. Es wurden 36 mg der Zielverbindung erhalten (33% d. Th.).
LC-MS (Methode 4): Rₜ = 4.41 min; MS (ESIpos): m/z = 399 (M+H)⁺
¹H-NMR (400 MHz, DMSO-d₆): δ [ppm] = 5.90 (s, 2H), 7.16 (t, 1H), 7.22-7.27 (m, 2H), 7.35-7.41 (m, 1H), 7.48 (dd, 1H), 7.87 (d, 2H), 8.72 (dd, 1H), 8.84 (d, 2H), 8.96 (dd, 1H).

### Beispiel 6

3-[1-(2-Fluorbenzyl)-1H-pyrazolo[3,4-b]pyridin-3-yl]-6-(1-methyl-1H-pyrazol-4-yl)-1,2,4-triazin-5-amin

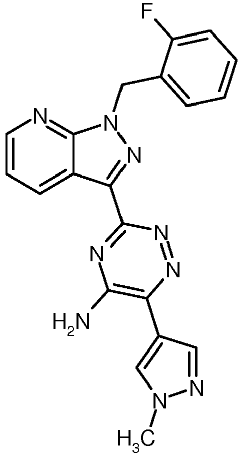

Unter einer Argonatmosphäre wurden 140 mg (Reinheit 65%, 0.256 mmol) 6-Chlor-3-[1-(2-fluorbenzyl)-1H-pyrazolo[3,4-b]pyridin-3-yl]-1,2,4-triazin-5-amin in 5 ml absolutem Dioxan suspendiert. Es wurden 160 mg (0.767 mmol) 1-Methyl-4-(4,4,5,5-tetramethyl-1,3,2-dioxaborolan-2-yl)-1H-pyrazol, 1.023 ml (1.023 mmol) 1N wässrige Kaliumcarbonat-Lösung sowie 14 mg (0.051 mmol) Tricyclohexylphosphin addiert und 10 min unter Rühren Argon durch die Suspension geleitet. Anschließend wurden 28 mg (0.038 mmol) 1,1'-Bis(diphenyl-phosphino)ferrocenpalladium(II)chlorid zugegeben und das Reaktionsgemisch für 30 min in der Mikrowelle bei 140°C gerührt. Es wurden erneut das Reaktionsgemisch für 15 min in der Mikrowelle bei 140°C gerührt. Nach dem Abkühlen wurde die Mischung über eine Extrelut-Kartusche filtriert und mit einem Gemische aus Dichlormethan/Methanol (v/v = 20:1) nachgewaschen. Das Filtrat wurde eingeengt und der Rückstand mittels präparativer HPLC (Eluent: Acetonitril/Wasser, Gradient 20:80 → 100:0) gereinigt. Es wurden 66 mg der Zielverbindung erhalten (47% d. Th.).
LC-MS (Methode 4): Rₜ = 4.40 min; MS (ESIpos): m/z = 402 (M+H)⁺
¹H-NMR (400 MHz, DMSO-d₆): δ [ppm] = 3.95 (s, 3H), 5.90 (s, 2H), 7.16 (dt, 1H), 7.22-7.27 (m, 2H), 7.35-7.41 (m, 1H), 7.49 (dd, 1H), 8.04 (s, 1H), 8.41 (s, 1H), 8.73 (dd, 1H), 8.96 (dd, 1H).

### Beispiel 7

### 3-[1-(2-Fluorbenzyl)-1H-pyrazolo[3,4-b]pyridin-3-yl]-6-(pyridin-3-yl)-1,2,4-triazin-5-amin

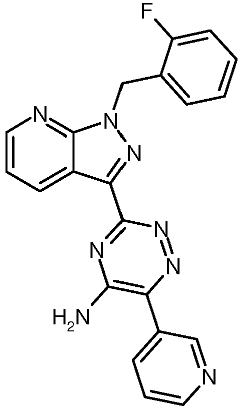

Unter einer Argonatmosphäre wurden 140 mg (Reinheit 65%, 0.256 mmol) 6-Chlor-3-[1-(2-fluorbenzyl)-1H-pyrazolo[3,4-b]pyridin-3-yl]-1,2,4-triazin-5-amin in 5 ml absolutem Dioxan suspendiert. Es wurden 94 mg (0.767 mmol) Pyridin-3-ylboronsäure, 1.023 ml (1.023 mmol) 1N wässrige Kaliumcarbonat-Lösung sowie 14 mg (0.051 mmol) Tricyclohexylphosphin addiert und 10 min unter Rühren Argon durch die Suspension geleitet. Anschließend wurden 28 mg (0.038 mmol) 1,1'-Bis(diphenylphosphino)ferrocenpalladium(II)chlorid zugegeben und das Reaktionsgemisch für 30 min in der Mikrowelle bei 140°C gerührt. Nach dem Abkühlen wurde die Mischung über eine Extrelut-Kartusche filtriert und mit einem Gemische aus Dichlormethan/Methanol (v/v = 20:1) nachgewaschen. Das Filtrat wurde eingeengt und der Rückstand mittels präparativer HPLC (Eluent: Acetonitril/Wasser, Gradient 20:80 → 100:0) gereinigt. Es wurden 77 mg der Zielverbindung erhalten (65% d. Th.).
LC-MS (Methode 4): Rₜ = 4.47 min; MS (ESIpos): m/z = 399 (M+H)⁺
¹H-NMR (400 MHz, DMSO-d₆): δ [ppm] = 5.92 (s, 2H), 7.17 (t, 1H), 7.22-7.27 (m, 2H), 7.36-7.41 (m, 1H), 7.51 (dd, 1H), 7.73 (dd, 1H), 8.27 (dt, 1H), 8.74 (dd, 1H), 8.82 (dd, 1H), 8.96 (dd, 2H).

### Beispiel 8

3-[1-(2-Fluorbenzyl)-1H-pyrazolo[3,4-b]pyridin-3-yl]-6-(2-methoxypyridin-3-yl)-1,2,4-triazin-5-amin

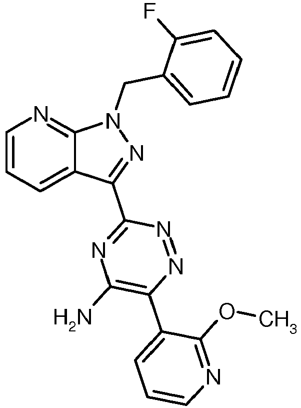

Unter einer Argonatmosphäre wurden 140 mg (Reinheit 65%, 0.256 mmol) 6-Chlor-3-[1-(2-fluorbenzyl)-1H-pyrazolo[3,4-b]pyridin-3-yl]-1,2,4-triazin-5-amin in 5 ml absolutem Dioxan suspendiert. Es wurden 117 mg (0.767 mmol) (2-Methoxypyridin-3-yl)boronsäure, 1.023 Il (1.023 nmlol) 1N wässrige Kaliumcarbonat-Lösung sowie 14 mg (0.051 mmol) Tricyclohexylphosphin addiert und 10 min unter Rühren Argon durch die Suspension geleitet. Anschließend wurden 28 mg (0.038 mmol) 1,1'-Bis(diphenylphosphino)ferrocenpalladium(II)chlorid zugegeben und das Reaktionsgemisch für 60 min in der Mikrowelle bei 140°C gerührt. Nach dem Abkühlen wurde die Mischung über eine Extrelut-Kartusche filtriert und mit einem Gemische aus Dichlormethan/Methanol (v/v = 20:1) nachgewaschen. Das Filtrat wurde eingeengt und der Rückstand mittels präparativer HPLC (Eluent: Acetonitril/Wasser, Gradient 20:80 →100:0) gereinigt. Es wurden 38 mg der Zielverbindung erhalten (27% d. Th.).
LC-MS (Methode 4): Rₜ = 4.72 min; MS (ESIpos): m/z = 429 (M+H)⁺
¹H-NMR (400 MHz, DMSO-d₆): δ [ppm] = 3.32 (s, 2H), 3.90 (s, 3H), 5.88 (s, 2H), 7.13-7.27 (m, 3H), 7.34-7.40 (m, 1H), 7.44 (dd, 1H), 7.53-7.65 (m, 1H), 7.87 (dd, 1H), 8.36 (dd, 1H), 8.69 (dd, 1H), 8.95 (dd, 1H).

### Beispiel 9

3-[1-(2-Fluorbenzyl)-1H-pyrazolo[3,4-b]pyridin-3-yl]-6-(2-methylpyridin-4-yl)-1,2,4-triazin-5-amin

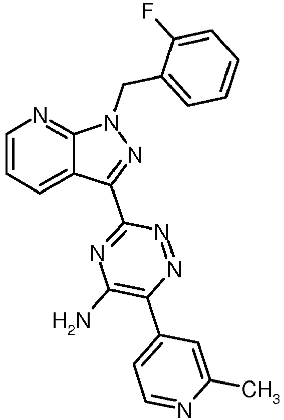

Unter einer Argonatmosphäre wurden 140 mg (Reinheit 65%, 0.256 mmol) 6-Chlor-3-[1-(2-fluorbenzyl)-1H-pyrazolo[3,4-b]pyridin-3-yl]-1,2,4-triazin-5-amin in 5 ml absolutem Dioxan suspendiert. Es wurden 105 mg (0.767 mmol) (2-Methylpyridin-4-yl)boronsäure, 1.023 ml (1.023 mmol) 1N wässrige Kaliumcarbonat-Lösung sowie 14 mg (0.051 mmol) Tricyclohexylphosphin addiert und 10 min unter Rühren Argon durch die Suspension geleitet. Anschließend wurden 28 mg (0.038 mmol) 1,1'-Bis(diphenylphosphino)ferrocenpalladium(II)chlorid zugegeben und das Reaktionsgemisch für 30 min in der Mikrowelle bei 140°C gerührt. Es wurden erneut 19 mg (0.026 mmol) 1,1'-Bis(diphenylphosphino)ferrocenpalladium(II)chlorid zugegeben und das Reaktionsgemisch für 40 min in der Mikrowelle bei 150°C gerührt. Nach dem Abkühlen wurde die Mischung über eine Extrelut-Kartusche filtriert und mit einem Gemische aus Dichlormethan/Methanol (v/v = 20:1) nachgewaschen. Das Filtrat wurde eingeengt und der Rückstand mittels präparativer HPLC (Eluent: Acetonitril/Wasser, Gradient 20:80 → 100:0) gereinigt. Es wurden 53 mg der Zielverbindung erhalten (Reinheit 92%, 41% d. Th.).
LC-MS (Methode 4): Rₜ = 4.25 min; MS (ESIpos): m/z = 413 (M+H)⁺
¹H-NMR (400 MHz, DMSO-d₆): δ [ppm] = 2.72 (s, 3H), 5.91 (s, 2H), 7.16 (t, 1H), 7.22-7.29 (m, 2H), 7.35-7.41 (m, 1H), 7.49 (dd, 1H), 7,93 (d, 1H), 8.01 (s, 1H), 8.72 (dd, 1H), 8.83 (d, 1H), 8.96 (dd, 1H).

### Beispiel 10

### 3-[1-(2-Fluorbenzyl)-1H-pyrazolo[3,4-b]pyridin-3-yl]-1,2,4-triazin-5,6-diamin

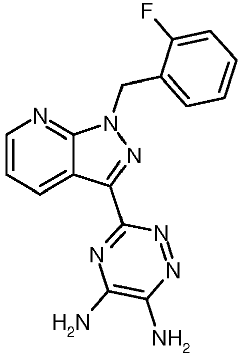

Es wurden 3.602 g (10.677 mmol) 6-Amino-3-[1-(2-fluorbenzyl)-1H-pyrazolo[3,4-b]pyridin-3-yl]-1,2,4-triazin-5-ol in 45 ml Thionylchlorid gegeben und 3h unter Rückfluss erhitzt. Die Reaktionsmischung wurde mit 200 ml trockenem Acetonitril verdünnt und unter Eiskühlung in 500 ml konzentrierte wässrige Anmloniak-Lösung (35%-ig) getropft. Das Gemische wurde über Nacht bei RT gerührt. Das Acetonitril wurde am Rotationsverdampfer entfernt und der Niederschlag abgesaugt. Es wurden 3.541 g (Reinheit 67%, 66% d. Th.) der Zielverbindung erhalten. Eine kleine Menge wurde mittels präparativer HPLC (Eluent: Acetonitril/Wasser mit 0.1% TFA, Gradient 30:70 →95:5) gereinigt.
LC-MS (Methode 1): Rₜ = 0.71 min; MS (ESIpos): m/z = 337 (M+H)⁺
¹H-NMR (400 MHz, DMSO-d₆): δ [ppm] = 5.90 (s, 2H), 7.13-7.18 (m, 1H), 7.21-7.27 (m, 2H), 7.35-7.41 (m, 1H), 7.45-7.53 (m, 3H), 8.53 (s br, 1H), 8.75 (dd, 1H), 8.84 (dd, 1H), 9.52 (s br, 1H).

### Beispiel 11

### N-{5-Amino-3-[1-(2-fluorbenzyl)-1H-pyrazolo[3,4-b]pyridin-3-yl]-1,2,4-triazin-6-yl}methansulfonamid

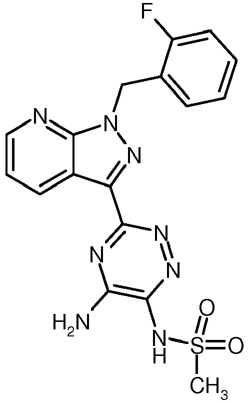

200 mg (0.595 mmol) 3-[1-(2-Fluorbenzyl)-1H-pyrazolo[3,4-b]pyridin-3-yl]-1,2,4-triazin-5,6-diamin wurden mit 8 ml Dichlormethan versetzt und auf 0°C gekühlt. Es wurden 545 mg 4.757 mmol) Methansulfonsäurechlorid sowie 481 mg (4.757 mmol) Trimethylamin addiert und das Gemisch bei RT für 72 h gerührt. Das Gemisch wurde mit Dichlormethan verdünnt und der Niederschlag abgesaugt. Das Filtrat wurde zwischen Wasser und Essigsäureethylester verteilt. Die organische Phase wurde über Natriumsulfat getrocknet und am Rotationsverdampfer eingeengt. Der Rückstand wurde mittels präparativer HPLC (Eluent: Acetonitril/Wasser mit 0.1% TFA, Gradient 30:70 → 95:5) gereinigt. Es wurden 57 mg (18% d. Th.) der Zielverbindung erhalten.
LC-MS (Methode 2): Rₜ = 0.77 min; MS (ESIpos): m/z = 415 (M+H)⁺
¹H-NMR (400 MHz, TFA-d₁): δ [ppm] = 3.47 (s, 3H), 6.05 (s, 2H), 7.08-7.14 (m, 1H), 7.29 (t, 1H), 7.44-7.50 (m, 1H), 7.54-7.60 (m, 1H), 8.09 (dd, 1H), 9.05-9.09 (m, 1H), 9.54 (dd, 1H).

### Beispiel 12

Methyl-{5-amino-3-[1-(2-fluorbenzyl)-1H-pyrazolo[3,4-b]pyridin-3-yl]-1,2,4-triazin-6-yl}carbamat

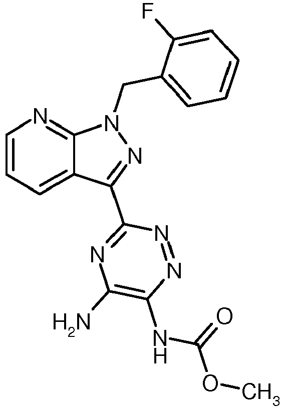

200 mg (Reinheit 67%, 0.398 mmol) 3-[1-(2-Fluorbenzyl)-1H-pyrazolo[3,4-b]pyridin-3-yl]-1,2,4-triazin-5,6-diamin wurden mit 4 ml Dichlormethan versetzt und auf 0°C gekühlt. Es wurden 151 mg 1.594 mmol) Chorameisensäuremethylester gelöst in 1 ml Dichlormethan sowie 161 mg (1.594 mmol) Triethylamin addiert und das Gemisch bei RT für 15 min gerührt. Der Niederschlag wurde abgesaugt und im Hochvakuum getrocknet. Es wurden 97 mg (61% d. Th.) der Zielverbindung erhalten.
LC-MS (Methode 5): Rₜ = 2.09 min; MS (ESIpos): m/z = 395 (M+H)⁺
¹H-NMR (400 MHz, DMSO-d₆): δ [ppm] = 4.00 (s, 3H), 5.79 (s, 2H), 7.11-7.16 (m, 2H), 7.20-7.25 (m, 1H), 7.32-7.40 (m, 2H), 8.00 (d br, 1H), 8.56 (d br, 1H), 8.64 (dd, 1H), 8.75 (dd, 1H), 9.86 (s, 1H).

### Beispiel 13

### 3-[1-(2-Fluorbenzyl)-1H-pyrazolo[3,4-b]pyridin-3-yl]-6-(trifluormethyl)-1,2,4-triazin-5-ol

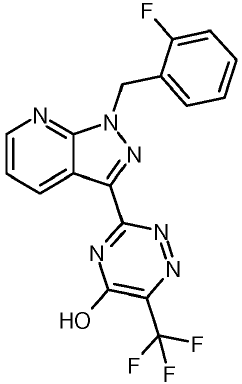

Es wurden 1.098 g (7.035 mmol) Methyl-3,3,3-trifluor-2-oxopropanoat in 10 ml Ethanol vorgelegt und zum Rückfluss erhitzt. Anschließend wurden 2.000 g (7.035 mmol) 1-(2-Fluorbenzyl)-1H-pyrazolo[3,4-d]pyrimidin-3-carboximidohydrazid suspendiert in 25 ml Ethanol addiert und über Nacht zum Rückfluss erhitzt. Nach dem Abkühlen wurde das Gemische filtriert, der Filterkuchen mit wenig Ethanol gewaschen und mittels präparativer HPLC (Eluent: Acetonitril/Wasser mit 0.1% TFA, Verhältnis 45:55) gereinigt. Es wurden 710 mg der Zielverbindung erhalten (Reinheit 93%, 24% d. Th.).
LC-MS (Methode 1) Rₜ = 0.97 min; MS (ESIpos): m/z = 391 (M+H)⁺
¹H-NMR (400 MHz, DMSO-d₆): δ [ppm] = 5.94 (s, 2H), 7.12-7.19 (m, 1H), 7.21-7.29 (m, 2H), 7.33-7.42 (m, 1H), 7.55 (dd, 1H), 8.73 (dd, 1H), 8.78 (dd, 1H).

### Beispiel 14

### 3-[1-(2-Fluorbenzyl)-1H-pyrazolo[3,4-b]pyridin-3-yl]-6-(trifluormethyl)-1,2,4-triazin-5-amin

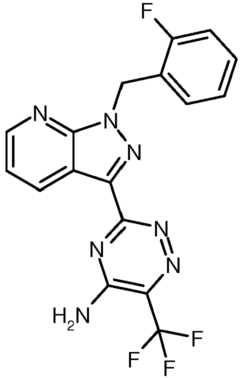

Es wurden 690 mg (1.768 mmol) 3-[1-(2-Fluorbenzyl)-1H-pyrazolo[3,4-b]pyridin-3-yl]-6-(trifluormethyl)-1,2,4-triazin-5-ol mit 9 ml Phosphorylchlorid versetzt und über Nacht bei RT gerührt. Das Reaktionsgemisch wurde mit 50 ml trockenem Acetonitril verdünnt und unter Eiskühlung in 123 ml konzentrierte wässrige Ammoniak-Lösung (25%-ig) eingerührt. Es wurde für 48h bei RT und 24h bei 50°C gerührt. Nach dem Abkühlen wurden das Acetonitril am Rotationsverdampfer entfernt, Wasser addiert, der Niederschlag abgesaugt und der Filterkuchen mit wenig Wasser gewaschen. Der Rückstand wurde mittels präparativer HPLC (Eluent: Acetonitril/Wasser mit 0.1% TFA, Gradient 30:70 → 95:5) gereinigt. Es wurden 125 mg (18% d. Th.) der Zielverbindung erhalten.
LC-MS (Methode 1) Rₜ = 1.02 min; MS (ESIpos): m/z = 390 (M+H)⁺
¹H-NMR (400 MHz, DMSO-d₆): δ [ppm] = 5.94 (s, 2H), 7.16 (t, 1H), 7.22-7.27 (m, 2H), 7.35-7.40 (m, 1H), 7.48 (dd, 1H), 7.79 (s br, 1H), 8.72 (dd, 1H), 8.78 (s br, 1H), 8.94 (dd, 1H).

### Beispiel 15

### 6-Cyclopentyl-3-[1-(2-fluorbenzyl)-1H-pyrazolo[3,4-b]pyridin-3-yl]-1,2,4-triazin-5-ol

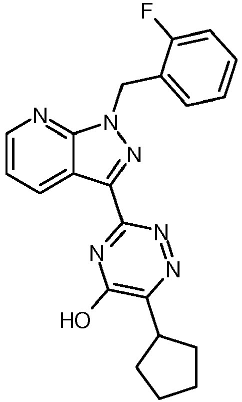

Es wurden 1.197 g (7.035 mmol) Ethylcyclopentyl(oxo)acetat in 15 ml Ethanol vorgelegt und zum Rückfluss erhitzt. Anschließend wurden 2.000 g (7.035 mmol) 1-(2-Fluorbenzyl)-1H-pyrazolo[3,4-d]pyrimidin-3-carboximidohydrazid suspendiert in 20 ml Ethanol addiert und über Nacht zum Rückfluss erhitzt. Nach dem Abkühlen wurde das Gemisch eingeengt und der Rückstand mittels präparativer HPLC (Eluent: Acetonitril/Wasser mit 0.1% TFA, Verhältnis 45:55) gereinigt. Es wurden 749 mg der Zielverbindung erhalten (26% d. Th.).

LC-MS (Methode 2) Rₜ = 1.10 min; MS (ESIpos): m/z = 391 (M+H)^{+ 1}H-NMR (400 MHz, DMSO-d₆): δ [ppm] = 1.60-1.78 (m, 6H), 1.91-1.98 (m, 2H), 5.90 (s, 2H), 7.15 (t, 1H), 7.22-7.28 (m, 2H), 7.35-7.40 (m, 1H), 7.48-7.51 (m, 1H), 8.73 (s, 1H), 8.75 (dd, 1H), 14.25 (s br, 1H).

### Beispiel 16

### 6-Cyclopentyl-3-[1-(2-fluorbenzyl)-1H-pyrazolo[3,4-b]pyridm-3-yl]-1,2,4-triazin-5-amin

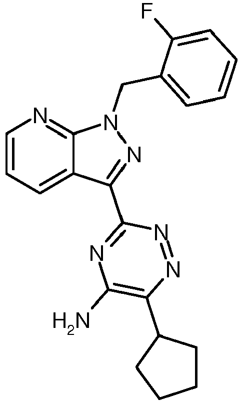

Es wurden 730 mg (1.870 mmol) 6-Cyclopentyl-3-[1-(2-fluorbenzyl)-1H-pyrazolo[3,4-b]pyridin-3-yl]-1,2,4-triazin-5-ol mit 9 ml Phosphorylchlorid versetzt und über Nacht bei RT gerührt. Das Reaktionsgemisch wurde mit 50 ml trockenem Acetonitril verdünnt und unter Eiskühlung in 130 ml konzentrierte wässrige Ammoniak-Lösung (25%-ig) eingerührt. Es wurde für 48h bei RT und 24h bei 50°C gerührt. Nach dem Abkühlen wurden das Acetonitril am Rotationsverdampfer entfernt, der Niederschlag abgesaugt und der Filterkuchen mit wenig Wasser gewaschen. Der Rückstand wurde mittels präparativer HPLC (Eluent: Acetonitril/Wasser mit 0.1% TFA, Gradient 30:70 → 95:5) gereinigt. Es wurden 508 mg (70% d. Th.) der Zielverbindung erhalten.
LC-MS (Methode 1) Rₜ = 0.90 min; MS (ESIpos): m/z = 390 (M+H)⁺
¹H-NMR (400 MHz, DMSO-d₆): δ [ppm] = 1.62-1.88 (m, 6H), 2.01-2.09 (m, 2H), 3.36 (qint, 1H), 5.92 (s, 2H), 7.16 (t, 1H), 7.22-7.29 (m, 2H), 7.35-7.41 (m, 1H), 7.54 (dd, 1H), 8.76 (dd, 1H), 8.90 (dd, 1H).

### Beispiel 17

### 3-[1-(2-Fluorbenzyl)-1H-pyrazolo[3,4-b]pyridin-3-yl]-6-propyl-1,2,4-triazin-5-ol

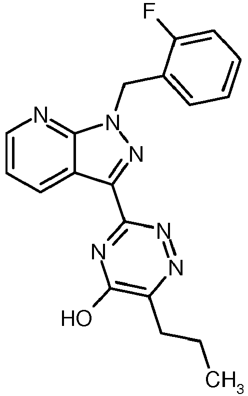

Es wurden 0.916 g (7.035 mmol) Methyl-2-oxopentanoat in 15 ml Ethanol vorgelegt und zum Rückfluss erhitzt. Anschließend wurden 2.000 g (7.035 mmol) 1-(2-Fluorbenzyl)-1H-pyrazolo[3,4-d]pyrimidin-3-carboximidohydrazid suspendiert in 20 ml Ethanol addiert und über Nacht zum Rückfluss erhitzt. Nach dem Abkühlen wurde das Gemisch abgesaugt, der Filterkuchen mit wenig Ethanol gewaschen und im Hochvakuum getrocknet. Es wurden 1.75 g der Zielverbindung erhalten (Reinheit 92%, 63% d. Th.).
LC-MS (Methode 1) Rₜ = 0.96 min; MS (ESIpos): m/z = 365 (M+H)⁺
¹H-NMR (400 MHz, DMSO-d₆): δ [ppm] = 0.95 (t, 3H), 1.66 (sext, 2H), 2.60 (t, 2H), 5.90 (s, 2H), 7.15 (t, 1H), 7.22-7.27 (m, 2H), 7.35-7.40 (m, 1H), 7.50 (dd, 1H), 8.73 (s, 1H), 8.75 (dd, 1H), 14.27 (s br, 1H).

### Beispiel 18

### 3-[1-(2-Fluorbenzyl)-1H-pyrazolo[3,4-b]pyridin-3-yl]-6-propyl-1,2,4-triazin-5-amin

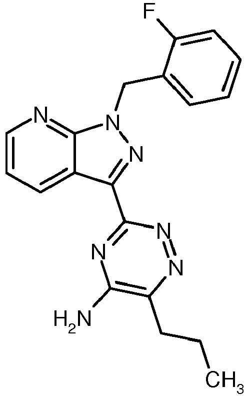

Es wurden 1.730 g (4.748 mmol) 3-[1-(2-Fluorbenzyl)-1H-pyrazolo[3,4-b]pyridin-3-yl]-6-propyl-1,2,4-triazin-5-ol mit 23 ml Phosphorylchlorid versetzt und über Nacht bei RT gerührt. Das Reaktionsgemisch wurde mit 100 ml trockenem Acetonitril verdünnt und unter Eiskühlung in 330 ml konzentrierte wässrige Ammoniak-Lösung (25%-ig) eingerührt. Es wurde für 48h bei RT und 24h bei 50°C gerührt. Nach dem Abkühlen wurden am Rotationsverdampfer eingeengt, der Rückstand mit 200 ml Wasser verrührt, abgesaugt und der Filterkuchen mit wenig Wasser gewaschen. Der Rückstand wurde mittels präparativer HPLC (Eluent: Acetonitril/Wasser mit 0.1% TFA, Gradient 30:70 → 95:5) gereinigt. Es wurden 1.360 g (60% d. Th.) der Zielverbindung erhalten.
LC-MS (Methode 1) Rₜ = 0.82 min; MS (ESIpos): m/z = 364 (M+H)⁺
¹H-NMR (400 MHz, DMSO-d₆): δ [ppm] = 1.00 (t, 3H), 1.72 (sext, 2H), 2.76 (t, 2H), 5.92 (s, 2H), 7.16 (t, 1H), 7.22-7.28 (m, 2H), 7.35-7.41 (m, 1H), 7.53 (dd, 1H), 8.76 (dd, 1H), 8.89 (dd, 1H).

### Beispiel 19

### 6-Ethyl-3-[1-(2-fluorbenzyl)-1H-pyrazolo[3,4-b]pyridin-3-yl]-1,2,4-triazin-5-ol

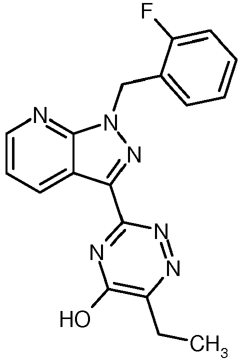

Es wurden 0.817 g (7.035 mmol) Methyl-2-oxobutanoat in 15 ml Ethanol vorgelegt und zum Rückfluss erhitzt. Anschließend wurden 2.000 g (7.035 mmol) 1-(2-Fluorbenzyl)-1H-pyrazolo[3,4-d]pyrimidin-3-carboximidohydrazid suspendiert in 20 ml Ethanol addiert und über Nacht zum Rückfluss erhitzt. Nach dem Abkühlen wurde das Gemisch abgesaugt, der Filterkuchen mit wenig Ethanol gewaschen und im Hochvakuum getrocknet. Es wurden 1.83 g der Zielverbindung erhalten (74% d. Th.).
LC-MS (Methode 5) Rₜ = 1.98 min; MS (ESIpos): m/z = 351 (M+H)⁺
¹H-NMR (400 MHz, DMSO-d₆): δ [ppm] = 1.17 (t, 3H), 2.66 (q, 2H), 5.90 (s, 2H), 7.15 (t, 1H), 7.22-7.27 (m, 2H), 7.35-7.41 (m, 1H), 7.50 (dd, 1H), 8.73 (s, 1H), 8.75 (d, 1H), 14.25 (s br, 1H).

### Beispiel 20

### 6-Ethyl-3-[1-(2-fluorbenzyl)-1H-pyrazolo[3,4-b]pyridin-3-yl]-1,2,4-triazin-5-amin

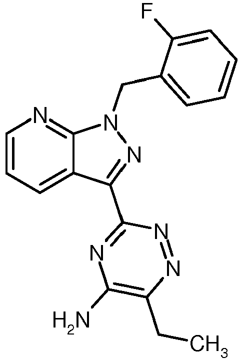

Es wurden 1.800 g (5.138 mmol) 6-Ethyl-3-[1-(2-fluorbenzyl)-1H-pyrazolo[3,4-b]pyridin-3-yl]-1,2,4-triazin-5-ol mit 25 ml Phosphorylchlorid versetzt und über Nacht bei RT gerührt. Das Reaktionsgemisch wurde mit 100 ml trockenem Acetonitril verdünnt und unter Eiskühlung in 375 ml konzentrierte wässrige Ammoniak-Lösung (25%-ig) eingerührt. Es wurde für 4h bei RT gerührt. Es wurde am Rotationsverdampfer eingeengt und der Rückstand zwischen Wasser und Essigsäureethylester verteilt. Die organische Phase wurde über Natriumsulfat getrocknet und am Rotationsverdampfer eingeengt. Der Rückstand wurde mittels präparativer HPLC (Eluent: Acetonitril/Wasser mit 0.1% TFA, Gradient 30:70 → 95:5) gereinigt. Es wurden 157 mg (8% d. Th.) der Zielverbindung erhalten.
LC-MS (Methode 1) Rₜ = 0.81 min; MS (ESIpos): m/z = 350 (M+H)⁺
¹H-NMR (400 MHz, DMSO-d₆): δ [ppm] = 1.26 (t, 3H), 2.80 (q, 2H), 5.92 (s, 2H), 7.16 (t, 1H), 7.22-7.29 (m, 2H), 7.35-7.41 (m, 1H), 7.54 (dd, 1H), 8.76 (dd, 1H), 8.89 (dd, 1H).

### Beispiel 21

### 3-[1-(2-Fluorbenzyl)-1H-pyrazolo[3,4-b]pyridin-3-yl]-6-isopropyl-1,2,4-triazin-5-ol

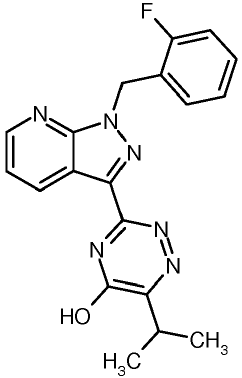

Es wurden 1.014 g (7.035 mmol) Ethyl-3-methyl-2-oxobutanoat in 15 ml Ethanol vorgelegt und zum Rückfluss erhitzt. Anschließend wurden 2.000 g (7.035 mmol) 1-(2-Fluorbenzyl)-1H-pyrazolo[3,4-d]pyrimidin-3-carboximidohydrazid suspendiert in 20 ml Ethanol addiert und über Nacht zum Rückfluss erhitzt. Nach dem Abkühlen wurde das Gemisch abgesaugt, der Filterkuchen mit wenig Ethanol gewaschen und im Hochvakuum getrocknet. Es wurden 918 mg der Zielverbindung erhalten (36% d. Th.).

LC-MS (Methode 1) Rₜ = 1.01 min; MS (ESIpos): m/z = 365 (M+H)^{+ 1}H-NMR (400 MHz, DMSO-d₆): δ [ppm] = 1.20 (d, 6H), 3.22 (sept, 1H), 5.90 (s, 2H), 7.15 (t, 1H), 7.22-7.27 (m, 2H), 7.35-7.40 (m, 1H), 7.49-7.52 (m, 1H), 8.73 (s, 1H), 8.75 (dd, 1H), 14.30 (s br, 1 H).

### Beispiel 22

### 3-[1-(2-Fluorbenzyl)-1H -pyrazolo[3,4-b]pyridin-3-yl]-6-isopropyl-1,2,4-triazin-5-amin

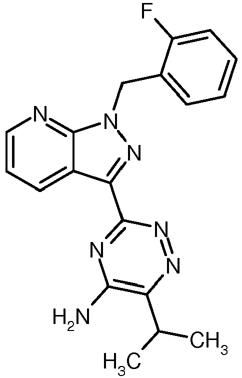

Es wurden 900 mg (5.138 mmol) 3-[1-(2-Fluorbenzyl)-1H-pyrazolo[3,4-b]pyridin-3-yl]-6-isopropyl-1,2,4-triazin-5-ol mit 12 ml Phosphorylchlorid versetzt und über Nacht bei RT gerührt. Das Reaktionsgemisch wurde mit 50 ml trockenem Acetonitril verdünnt und unter Eiskühlung in 173 ml konzentrierte wässrige Ammoniak-Lösung (25%-ig) eingerührt. Das Reaktionsgemisch wurde für 2h bei RT und 6h bei 50°C gerührt. Nach dem Abkühlen wurden am Rotationsverdampfer eingeengt. Der Rückstand wurde zwischen Wasser und Essigsäureethylester verteilt. Die organische Phase wurde über Natriumsulfat getrocknet und am Rotationsverdampfer eingeengt. Der Rückstand wurde mit Diethylether verrührt, abgesaugt und im Hochvakuum getrocknet. Es wurden 266 mg (Reinheit 92%, 27% d. Th.) der Zielverbindung erhalten.
LC-MS (Methode 2) Rₜ = 0.82 min; MS (ESIpos): m/z = 364 (M+H)⁺
¹H-NMR (400 MHz, DMSO-d₆): δ [ppm] = 1.28 (d, 6H), 3.24 (sept, 1H), 5.84 (s, 2H), 7.12-7.26 (m, 3H), 7.33-7.39 (m, 1H), 7.41 (dd, 1H), 8.66 (d, 1H), 8.91 (d, 1H).

### Beispiel 23

### 3-[1-(2-Fluorbenzyl)-1H-pyrazolo[3,4-b]pyridin-3-yl]-6-(1-fluorethyl)-1,2,4-triazin-5 -amin

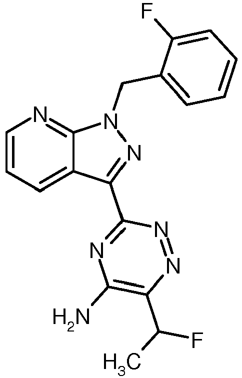

290 mg (Reinheit 95%, 0.671 mmol) 2-{5-Amino-3-[1-(2-fluorbenzyl)-1H-pyrazolo[3,4-b]pyridin-3-yl]-1,2,4-triazin-6-yl}-2-fluorpropanamid wurden in 2.6 ml Essigsäure vorgelegt und mit 2 Tropfen 1N Salzsäure versetzt. Das Gemisch wurde 30 min bei 100°C in der Mikrowelle gerührt. Nach dem Abkühlen wurde die Reaktionslösung mit Essigsäureethylester versetzt und mit gesättigter Natriumhydrogencarbonat-Lösung gewaschen. Die organische Phase wurde über Natriumsulfat getrocknet und eingeengt. Der Rückstand wurde mittels präparativer HPLC (Eluent: Acetonitril/Wasser, Gradient 30:70 → 95:5) gereinigt. Es wurden 23 mg (Reinheit 89%, 8% d. Th.) der Zielverbindung erhalten.
LC-MS (Methode 1) Rₜ = 0.88 min; MS (ESIpos): m/z = 368 (M+H)⁺
¹H-NMR (400 MHz, DMSO-d₆): δ [ppm] = 1.76 (dd, 3H), 5.86 (s, 2H), 6.03 (dq, 1H), 7.13-7.26 (m, 3H), 7.34-7.40 (m, 1H), 7.44 (dd, 1H), 7.50 (s br, 1H), 8.24 (s br, 1H), 8.69 (dd, 1H), 8.92 (dd, 1H).

### B. Bewertung der pharmakologischen Wirksamkeit

Die pharmakologische Wirkung der erfindungsgemäßen Verbindungen kann in folgenden Assays gezeigt werden:

### B-1. Gefäßrelaxierende Wirkung in vitro

Kaninchen werden durch Nackenschlag betäubt und entblutet. Die Aorta wird entnommen, von anhaftendem Gewebe befreit, in 1.5 mm breite Ringe geteilt und einzeln unter einer Vorspannung in 5 ml-Organbäder mit 37°C warmer, Carbogen-begaster Krebs-Henseleit-Lösung folgender Zusammensetzung gebracht (jeweils mM): Natriumchlorid: 119; Kaliumchlorid: 4.8; Calciumchlorid-Dihydrat: 1; Magnesiumsulfat-Heptahydrat: 1.4; Kaliumdihydrogenphosphat: 1.2; Natriumhydrogencarbonat: 25; Glucose: 10. Die Kontraktionskraft wird mit Statham UC2-Zellen erfasst, verstärkt und über A/D-Wandler (DAS-1802 HC, Keithley Instruments München) digitalisiert sowie parallel auf Linienschreiber registriert. Zur Erzeugung einer Kontraktion wird Phenylephrin dem Bad kumulativ in ansteigender Konzentration zugesetzt. Nach mehreren Kontrollzyklen wird die zu untersuchende Substanz in jedem weiteren Durchgang in jeweils steigender Dosierung zugesetzt und die Höhe der Kontraktion mit der Höhe der im letzten Vordurchgang erreichten Kontraktion verglichen. Daraus wird die Konzentration errechnet, die erforderlich ist, um die Höhe des Kontrollwertes um 50% zu reduzieren (IC₅₀-Wert). Das Standardapplikationsvolumen beträgt 5 µl, der DMSO-Anteil in der Badlösung entspricht 0.1%.

Repräsentative IC₅₀-Werte für die erfindungsgemäßen Verbindungen sind in der nachstehenden Tabelle (Tabelle 1) wiedergegeben:

**Tabelle 1:**

| Beispiel Nr. | IC₅₀ [nM] |
|---|---|
| 11 | 2960 |
| 12 | 112 |
| 13 | 2630 |
| 14 | 52 |
| 15 | 1090 |
| 16 | 25 |
| 18 | 30 |
| 20 | 30 |
| 21 | 916 |
| 22 | 27 |
| 23 | 23 |

### B-2. Wirkung an rekombinanter Guanylatcyclase-Reporterzelllinie

Die zelluläre Wirkung der erfindungsgemäßen Verbindungen wird an einer rekombinanten Guanylatcyclase-Reporterzelllinie, wie in F. Wunder et al., Anal. Biochem. 339, 104-112 (2005) beschrieben, bestimmt.

Repräsentative Werte (MEC = minimal effektive Konzentration) für die erfindungsgemäßen Verbindungen sind in der nachstehenden Tabelle (Tabelle 2) wiedergegeben:

**Tabelle 2:**

| Beispiel Nr. | MEC [µM] |
|---|---|
| 1 | 0.1 |
| 2 | 0.1 |
| 3 | 0.3 |
| 4 | 0.03 |
| 5 | 0.1 |
| 6 | 0.1 |
| 7 | 0.1 |
| 8 | 0.1 |
| 9 | 0.1 |
| 10 | 0.1 |
| 11 | 1.0 |
| 12 | 0.3 |
| 13 | 3.0 |
| 14 | 0.03 |
| 15 | 1.0 |
| 16 | 0.03 |
| 17 | 1.0 |
| 18 | 0.1 |
| 19 | 1.0 |
| 20 | 0.1 |
| 21 | 0.3 |
| 22 | 0.1 |
| 23 | 0.1 |

### B-3. Radiotelemetrische Blutdruckmessung an wachen, spontan hypertensiven Ratten

Für die im Folgenden beschriebene Blutdruckmessung an wachen Ratten wird ein im Handel erhältliches Telemetriesystem der Firma DATA SCIENCES INTERNATIONAL DSI, USA eingesetzt.

Das System besteht aus 3 Hauptkomponenten:
- Implantierbare Sender (Physiotel® Telemetrietransmitter)
- Empfänger (Physiotel® Receiver), die über einen Multiplexer (DSI Data Exchange Matrix) mit einem
- Datenakquisitionscomputer

### verbunden sind.

Die Telemetrieanlage ermöglicht eine kontinuierliche Erfassung von Blutdruck Herzfrequenz und Körperbewegung an wachen Tieren in ihrem gewohnten Lebensraum.

### Tiermaterial

Die Untersuchungen werden an ausgewachsenen weiblichen spontan hypertensiven Ratten (SHR Okamoto) mit einem Körpergewicht von >200 g durchgeführt. SHR/NCrl von Okamoto Kyoto School of Medicine, 1963 wurden aus männlichen Wistar Kyoto Ratten mit stark erhöhtem Blutdruck und weiblichen mit leicht erhöhtem Blutdruck gekreuzt und in der F13 an die U.S. National Institutes of Health abgegeben.

Die Versuchstiere werden nach Senderimplantation einzeln in Makrolon - Käfigen Typ 3 gehalten. Sie haben freien Zugang zu Standardfutter und Wasser.

Der Tag - Nacht - Rhythmus im Versuchslabor wird per Raumbeleuchtung um 6:00 Uhr morgens und um 19:00 Uhr abends gewechselt.

### Senderimplantation

Die eingesetzten Telemetriesender TA11 PA - C40 werden den Versuchstieren mindestens 14 Tage vor dem ersten Versuchseinsatz unter aseptischen Bedingungen chirurgisch implantiert. Die so instrumentierten Tiere sind nach Abheilen der Wunde und Einwachsen des Implantats wiederholt einsetzbar.

Zur Implantation werden die nüchternen Tiere mit Pentobabital (Nembutal, Sanofi: 50mg/kg i.p.) narkotisiert und an der Bauchseite weiträumig rasiert und desinfiziert. Nach Eröffnung des Bauchraumes entlang der Linea alba wird der flüssigkeitsgefüllte Meßkatheter des Systems oberhalb der Bifurcation nach cranial in die Aorta descendens eingesetzt und mit Gewebekleber (VetBonD TM, 3M) befestigt. Das Sendergehäuse wird intraperitoneal an der Bauchwandmuskulatur fixiert und die Wunde wird schichtweise verschlossen.

### Postoperativ wird zur Infektionsprophylaxe ein Antibiotikum verabreicht (Tardomyocel COMP Bayer 1ml/kg s.c.)

### Substanzen und Lösungen

Wenn nicht anders beschrieben werden die zu untersuchenden Substanzen jeweils einer Gruppe von Tieren (n = 6) per Schlundsonde oral verabreicht. Entsprechend einem Applikationsvolumen von 5 ml/kg Körpergewicht werden die Testsubstanzen in geeigneten Lösungsmittelgemischen gelöst oder in 0.5%-iger Tylose suspendiert.

Eine Lösungsmittel- behandelte Gruppe von Tieren wird als Kontrolle eingesetzt.

### Versuchsablauf

Die vorhandene Telemetrie - Meßeinrichtung ist für 24 Tiere konfiguriert. Jeder Versuch wird unter einer Versuchsnummer registiert (VJahr Monat Tag).

Den in der Anlage lebenden instrumentierten Ratten ist jeweils eine eigene Empfangsantenne zugeordnet (1010 Receiver, DSI).

Die implantierten Sender sind über einen eingebauten Magnetschalter von außen aktivierbar. Sie werden bei Versuchsvorlauf auf Sendung geschaltet. Die ausgestrahlten Signale können durch ein Datenakquisitionssystem (Dataquest TM A.R.T. for WINDOWS, DSI) online erfasst und entsprechend aufgearbeitet werden. Die Ablage der Daten erfolgt jeweils in einem hierfür eröffneten Ordner der die Versuchsnummer trägt.

Im Standardablauf werden über je 10 Sekunden Dauer gemessen:
- Systolischer Blutdruck (SBP)
- Diastolischer Blutdruck (DBP)
- Arterieller Mitteldruck (MAP)
- Herzfrequenz (HR)
- Aktivität (ACT).

Die Messwerterfassung wird rechnergesteuert in 5 Minuten Abständen wiederholt. Die als Absolutwert erhobenen Quelldaten werden im Diagramm mit dem aktuell gemessenen Barometerdruck (Ambient Pressure Reference Monitor; APR-1) korrigiert und in Einzeldaten abgelegt. Weitere technische Details sind der umfangreichen Dokumentation der Herstellerfirma (DSI) zu entnehmen.

Wenn nicht anders beschrieben erfolgt die Verabreichung der Prüfsubstanzen am Versuchstag um 9.00 Uhr. Im Anschluss an die Applikation werden die oben beschriebenen Parameter 24 Stunden gemessen.

### Auswertung

Nach Versuchsende werden die erhobenen Einzeldaten mit der Analysis-Software (DATAQUEST TM A. R.T. TM ANALYSIS) sortiert. Als Leerwert werden hier 2 Stunden vor Applikation angenommen, so dass der selektierte Datensatz den Zeitraum von 7:00 Uhr am Versuchstag bis 9:00 Uhr am Folgetag umfasst.

Die Daten werden über eine voreinstellbare Zeit durch Mittelwertbestimmung geglättet (15 Minuten Average) und als Textdatei auf einen Datenträger übertragen. Die so vorsortierten und komprimierten Messwerte werden in Excel-Vorlagen übertragen und tabellarisch dargestellt. Die Ablage der erhobenen Daten erfolgt pro Versuchstag in einem eigenen Ordner, der die Versuchsnummer trägt. Ergebnisse und Versuchsprotokolle werden in Papierform nach Nummern sortiert in Ordnern abgelegt.

### Literatur

Klaus Witte, Kai Hu, Johanna Swiatek, Claudia Müssig, Georg Ertl and Björn Lemmer: Experimental heart failure in rats: effects on cardiovascular circadian rhythms and on myocardial β-adrenergic signaling. Cardiovasc Res 47 (2): 203-405, 2000; Kozo Okamoto: Spontaneous hypertension in rats. Int Rev Exp Pathol 7: 227- 270, 1969; Maarten van den Buuse: Circadian Rhythms of Blood Pressure, Heart Rate, and Locomotor Activity in Spontaneously Hypertensive Rats as Measured With Radio-Telemetry. Physiology & Behavior 55(4): 783-787, 1994

### B-4. Bestimmung pharmakokinetischer Kenngrößen nach intravenöser und oraler Gabe

Die pharmakokinetischen Parameter der erfindungsgemäßen Verbindungen werden in männlichen CD-1-Mäusen, männlichen Wister-Ratten und weiblichen Beagle-Hunden bestimmt. Die intravenöse Gabe erfolgt bei Mäusen und Ratten mittels einer speziesspezifischen Plasma/DMSO-Formulierung und bei Hunden mittels einer Wasser/PEG400/Ethanol-Formulierung. Die orale Gabe der gelösten Substanz mittels Schlundsonde wird in allen Spezies basierend auf einer Wasser/PEG400/Ethanol-Formulierung durchgeführt. Den Ratten wird zur vereinfachten Blutabnahme vor der Substanzgabe ein Silikonkatheter in die rechte *Vena jugularis externa* gelegt. Die Operation erfolgt mindestens einen Tag vor dem Versuch unter Isofluran-Narkose und unter Gabe eines Analgetikums (Atropin/Rimadyl (3/1) 0.1 mL s.c.). Die Blutabnahme (in der Regel mehr als 10 Zeitpunkte) erfolgt in einem Zeitfenster, welches terminale Zeitpunkte von mindestens 24 bis maximal 72 Stunden nach Substanzgabe beinhaltet. Das Blut wird bei der Entnahme in heparinisierte Röhrchen geleitet. So dann wird mittels Zentrifugation das Blutplasma gewonnen und gegebenenfalls bis zur weiteren Bearbeitung bei -20°C gelagert.

Den Proben der erfindungsgemäßen Verbindungen, Kalibrierproben und Qualifier wird ein interner Standard zugesetzt (dies kann auch eine chemisch nicht verwandte Substanz sein) und es folgt eine Proteinfällung mittels Acetonitril im Überschuss. Nach Zugabe einer Puffer-Lösung, die an die LC-Bedingungen angepasst ist, und folgendem Vortexen wird bei 1000 g zentrifugiert. Der Überstand wird mittels LC-MS/MS unter Verwendung von C18-reversed-phase-Säulen und variablen Eluenten-Gemischen vermessen. Die Quantifizierung der Substanzen erfolgt anhand der Peakhöhen oder -flächen aus extrahierten Ionenchromatogrammen spezifischer selected ion monitoring-Experimente.

Aus den ermittelten Plasmakonzentration-Zeit-Verläufen werden die pharmakokinetischen Kenngrößen wie AUC, Cₘₐₓ, t_{1/2} (terminale Halbwertszeit), MRT (Mean Residence Time) und CL (Clearance) mittels eines validierten pharmakokinetischen Rechenprogramms berechnet.

Da die Substanzquantifizierung in Plasma durchgeführt wird, muss die Blut/Plasma-Verteilung der Substanz bestimmt werden, um die pharmakokinetischen Parameter entsprechend anpassen zu können. Dazu wird eine definierte Menge Substanz in heparinisiertem Vollblut der entsprechenden Spezies für 20 min im Taumelrollenmischer inkubiert. Nach Zentrifugation bei 1000g wird die Konzentration im Plasma gemessen (mittels LC-MS/MS; s.o.) und durch Quotientenbildung der C_{Blut}/C_{Plasma}-Wert vermittelt.

### B-5. Metabolismus-Untersuchung

Zur Bestimmung des Metabolismus-Profils der erfindungsgemäßen Verbindungen werden diese mit rekombinanten humanen Cytochrom P450 (CYP) Enzymen, Lebermikrosomen oder mit primären frischen Hepatozyten verschiedener Tierspezies (z.B. Ratte, Hund) als auch humanen Ursprungs inkubiert, um Informationen über einen möglichst kompletten hepatischen Phase I- und Phase II-Metabolismus sowie über die am Metabolismus beteiligten Enzyme zu erhalten und zu vergleichen.

Die erfindungsgemäßen Verbindungen wurden mit einer Konzentration von etwa 0.1-10 µM inkubiert. Dazu wurden Stammlösungen der erfindungsgemäßen Verbindungen mit einer Konzentration von 0.01-1 mM in Acetonitril hergestellt, und dann mit einer 1:100 Verdünnung in den Inkubationsansatz pipettiert. Die Lebermikrosomen und rekombinanten Enzyme wurden in 50 mM Kaliumphosphatpuffer pH 7.4 mit und ohne NADPH-generierendem System, bestehend aus 1 mM NADP⁺, 10 mM Glucose-6-phosphat und 1 Unit Glucose-6-phosphat Dehydrogenase, bei 37°C inkubiert. Primäre Hepatozyten wurden in Suspension in Williams E Medium ebenfalls bei 37°C inkubiert. Nach einer Inkubationszeit von 0 - 4h wurden die Inkubationsansätze mit Acetonitril abgestoppt (Endkonzentration ca. 30%) und das Protein bei ca. 15000 x g abzentrifugiert. Die so abgestoppten Proben wurden entweder direkt analysiert oder bis zur Analyse bei -20°C gelagert.

Die Analyse erfolgt mittels Hochleistungsflüssigkeits-Chromatographie mit Ultraviolett- und massenspektrometrischer Detektion (HPLC-UV-MS/MS). Dazu werden die Überstände der Inkubationsproben mit geeigneten C18-reversed-phase-Säulen und variablen Eluenten-Gemischen aus Acetonitril und 10 mM wässriger Ammoniumformiat-Lösung oder 0.05 % Ameisensäure chromatographiert. Die UV-Chromatogramme in Verbindung mit massenspektrometrischen Daten dienen zur Identifizierung, Strukturaufklärung und quantitativen Abschätzung der Metabolite, und der quantitativen metabolischen Abnahme der erfindungsgemäßen Verbindung in den Inkubationsansätzen.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

### Substituierte annellierte Pyrimidine und ihre Verwendung

### Zusammenfassung

Die vorliegende Anmeldung betrifft neue substituierte annellierte Pyrimidine, Verfahren zu ihrer Herstellung, ihre Verwendung allein oder in Kombinationen zur Behandlung und/oder Prophylaxe von Krankheiten sowie ihre Verwendung zur Herstellung von Arzneimitteln zur Behandlung und/oder Prophylaxe von Krankheiten, insbesondere zur Behandlung und/oder Prophylaxe von Herz-Kreislauf-Erkrankungen.

## Patentansprüche

1. Verbindung der Formel (I) 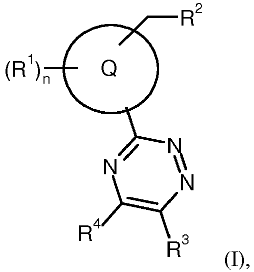 in welcher
der Ring Q für eine Gruppe der Formel 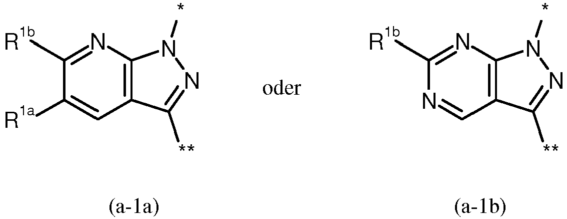 steht, wobei
* für die Anknüpfungsstelle an -CH₂-R² steht,
** für die Anknüpfungsstelle an den Triazinring steht,
R^{1a} für Wasserstoff oder Fluor steht,
R^{1b} für Wasserstoff oder Methyl steht,
R² für 3,3,3-Trifluorprop-1-yl, 2,2,3,3-Tetrafluorprop-1-yl, 2,2,3,3,3-Pentafluorprop-1-yl, Phenyl oder Pyridyl steht, wobei Phenyl mit 1 bis 3 Substituenten Fluor substituiert ist, und wobei Pyridyl mit 1 Substituenten Fluor substituiert sein kann,
R³ für Difluormethyl, Trifluormethyl, (C₁-C₆)-Alkyl, Cyclopropyl, Cyclobutyl, Cyclopentyl, Methylsulfonylamino, Methoxycarbonylamino, Phenyl, Pyrazolyl, Oxazolyl oder Pyridyl steht, wobei (C₁-C₆)-Alkyl mit 1 bis 3 Substituenten unabhängig voneinander ausgewählt aus der Gruppe Fluor, Trifluormethyl, Cyclopropyl, Cyclobutyl, Cyclopentyl, Difluormethoxy, Trifluormethoxy, Methoxy und Ethoxy substituiert sein können, und wobei Phenyl, Pyrazolyl, Oxazolyl und Pyridyl mit 1 oder 2 Substituenten unabhängig voneinander ausgewählt aus der Gruppe Fluor, Chlor, Difluormethyl, Trifluormethyl, Methyl, Ethyl, Cyclopropyl, Cyclobutyl, Cyclopentyl, Trifluormethoxy, Methoxy und Ethoxy substituiert sein können,
R⁴ für Hydroxy oder Amino steht,
sowie ihre Salze, Solvate und Solvate der Salze.

2. Verfahren zur Herstellung von Verbindungen der Formel (I), wie in Anspruch 1 definiert, **dadurch gekennzeichnet, dass** man
[A] eine Verbindung der Formel (II) 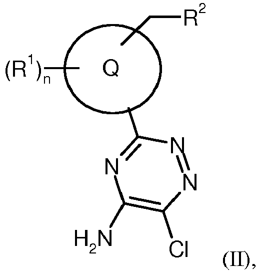 in welcher n, Q, R¹ und R² jeweils die in Anspruch 1 genannten Bedeutungen haben,
in einem inerten Lösungsmittel in Gegenwart eines geeigneten Übergangsmetallkatalysators mit einer Verbindung der Formel (III) 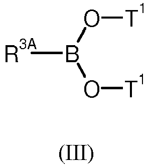 in welcher
R^{3A} für Phenyl oder 5- oder 6-gliedriges Heteroaryl steht, wobei Phenyl und 5- oder 6-gliedriges Heteroaryl mit 1 bis 3 Substituenten unabhängig voneinander ausgewählt aus der Gruppe Halogen, Trifluormethyl, (C₁-C₄)-Alkyl, (C₃-C₇)-Cycloalkyl, Difluormethoxy, Trifluormethoxy und (C₁-C₆)-Alkoxy substituiert sein können,
und
T¹ für Wasserstoff oder (C₁-C₄)-Alkyl steht, oder beide Reste R¹¹ zusammen eine -C(CH₃)₂-C(CH₃)₂-Brücke bilden,
zu einer Verbindung der Formel (I-A) 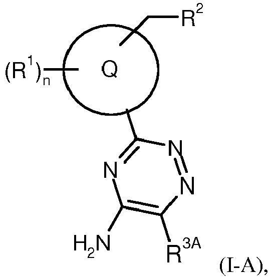 in welcher n, Q, R¹, R² und R^{3A} jeweils die in Anspruch 1 angegebenen Bedeutungen haben,
umsetzt,
oder
[B] eine Verbindung der Formel (IV) 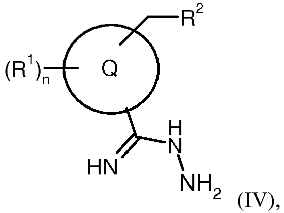 in welcher n, Q, R¹ und R² jeweils die in Anspruch 1 angegebenen Bedeutungen haben,
in einem inerten Lösungsmittel mit einer Verbindung der Formel (V) 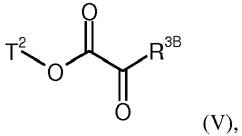 in welcher
R^{3B} für Difluormethyl, Trifluormethyl, (C₁-C₆)-Alkyl oder (C₃-C₇)-Cycloalkyl steht, wobei (C₁-C₆)-Alkyl mit 1 bis 3 Substituenten unabhängig voneinander ausgewählt aus der Gruppe Halogen, Trifluormethyl, (C₁-C₄)-Alkyl, (C₃-C₇)-Cycloalkyl, Difluormethoxy, Trifluormethoxy und (C₁-C₆)-Alkoxy substituiert sein kann, und
T² für (C₁-C₄)-Alkyl steht,
zu einer Verbindung der Formel (I-B) 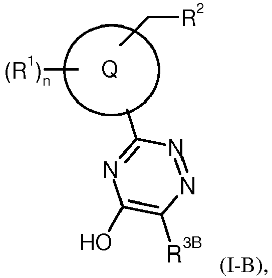 in welcher n, Q, R¹, R² und R^{3B} jeweils die zuvor angegebenen Bedeutungen haben,
umsetzt,
oder
[C] eine Verbindung der Formel (I-B) mit Phosphorylchlorid in eine Verbindung der Formel (VI) 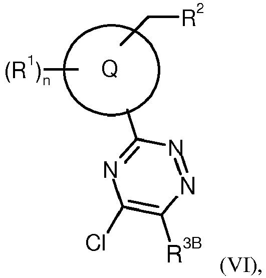 in welcher n, Q, R¹, R² und R^{3B} jeweils die zuvor angegebenen Bedeutungen haben,
überführt, und diese direkt mit Ammoniak zu einer Verbindung der Formel (I-C) 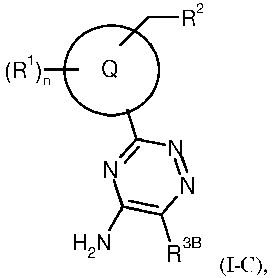 in welcher n, Q, R¹, R² und R^{3B} jeweils die zuvor angegebenen Bedeutungen haben,
umsetzt,
und gegebenenfalls die resultierenden Verbindungen der Formel (I-A), (I-B) und (I-C) gegebenenfalls mit den entsprechenden (i) Lösungsmitteln und/oder (ii) Säuren oder Basen in ihre Solvate, Salze und/oder Solvate der Salze überführt.

3. Verbindung der Formel (I), wie in Anspruch 1 definiert, zur Behandlung und/oder Prophylaxe von Krankheiten.

4. Verwendung einer Verbindung der Formel (I), wie in Anspruch 1 definiert, zur Herstellung eines Arzneimittels zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

5. Arzneimittel enthaltend eine Verbindung der Formel (I), wie in Anspruch 1 definiert, in Kombination mit einem inerten, nicht-toxischen, pharmazeutisch geeigneten Hilfsstoff.

6. Arzneimittel enthaltend eine Verbindung der Formel (I), wie in Anspruch 1 definiert, in Kombination mit einem weiteren Wirkstoff ausgewählt aus der Gruppe bestehend aus organischen Nitraten, NO-Donatoren, cGMP-PDE-Inhibitoren, antithrombotisch wirkenden Mitteln, den Blutdruck senkenden Mitteln sowie den Fettstoffwechsel verändernden Mitteln.

7. Arzneimittel nach Anspruch 5 oder 6 zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

## Claims

1. Compound of the formula (I) 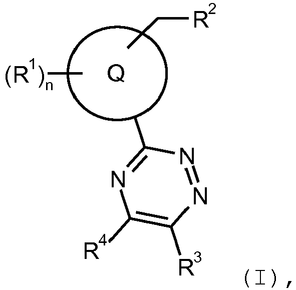 in which
the ring Q is a group of the formula 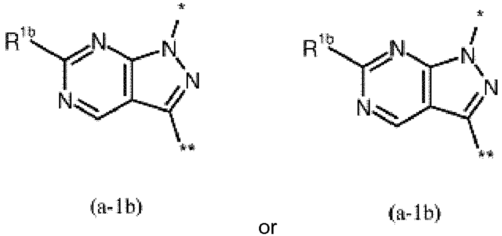 where
* is the attachment site to -CH₂-R²,
** is the attachment site to the triazine ring,
R^{1a} is hydrogen or fluorine,
R^{1b} is hydrogen or methyl,
R² is 3,3,3-trifluoroprop-1-yl, 2,2,3,3-tetrafluoroprop-1-yl, 2,2,3,3,3-pentafluoroprop-1-yl, phenyl or pyridyl, where phenyl is substituted by 1 to 3 fluorine substituents, and where pyridyl may be substituted by 1 fluorine substituent,
R³ is difluoromethyl, trifluoromethyl, (C₁-C₆)-alkyl, cyclopropyl, cyclobutyl, cyclopentyl, methylsulphonylamino, methoxycarbonylamino, phenyl, pyrazolyl, oxazolyl or pyridyl, where (C₁-C₆)-alkyl may be substituted by 1 to 3 substituents independently of one another selected from the group consisting of fluorine, trifluoromethyl, cyclopropyl, cyclobutyl, cyclopentyl, difluoromethoxy, trifluoromethoxy, methoxy and ethoxy, and where phenyl, pyrazolyl, oxazolyl and pyridyl may be substituted by 1 or 2 substituents independently of one another selected from the group consisting of fluorine, chlorine, difluoromethyl, trifluoromethyl, methyl, ethyl, cyclopropyl, cyclobutyl, cyclopentyl, trifluoromethoxy, methoxy and ethoxy,
R⁴ is hydroxy or amino, and the salts, solvates and solvates of the salts thereof.

2. Process for preparing compounds of the formula (I) as defined in Claim 1, **characterized in that**
[A] a compound of the formula (II) 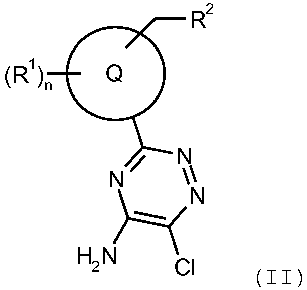 in which n, Q, R¹ and R² each have the meanings specified in Claim 1,
is reacted in an inert solvent in the presence of a suitable transition metal catalyst with a compound of the formula (III) 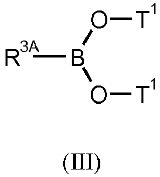 in which
R^{3A} is phenyl or 5- or 6-membered heteroaryl, where phenyl and 5- or 6-membered heteroaryl may be substituted by 1 to 3 substituents independently of one another selected from the group consisting of halogen, trifluoromethyl, (C₁-C₄)-alkyl, (C₃-C₇) -cycloalkyl, difluoromethoxy, trifluoromethoxy and (C₁-C₆)-alkoxy,
and
T¹ is hydrogen or (C₁-C₄) -alkyl, or both R¹¹ radicals together form a -C(CH₃)₂-C(CH₃)₂-bridge,
to give a compound of the formula (I-A) 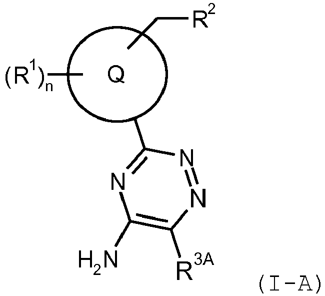 in which n, Q, R¹, R² and R^{3A} each have the meanings specified in Claim 1,
or
[B] a compound of the formula (IV) 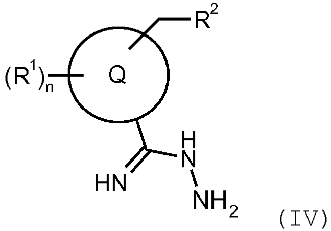 in which n, Q, R¹ and R² each have the meanings specified in Claim 1, is reacted in an inert solvent with a compound of the formula (V) 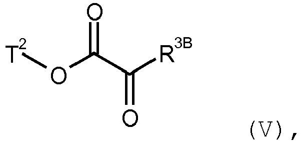 in which
R^{3B} is difluoromethyl, trifluoromethyl, (C₁-C₆)-alkyl or (C₃-C₇)-cycloalkyl, where (C₁-C₆)-alkyl may be substituted by 1 to 3 substituents independently of one another selected from the group consisting of halogen, trifluoromethyl, (C₁-C₄)-alkyl, (C₃-C₇)-cycloalkyl, difluoromethoxy, trifluoromethoxy and (C₁-C₆)-alkoxy, and
T² is (C₁-C₄)-alkyl,
to give a compound of the formula (I-B) 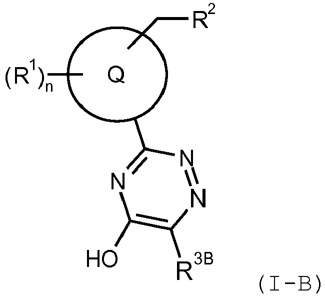 in which n, Q, R¹, R² and R^{3B} each have the meanings specified above,
or
[C] a compound of the formula (I-B) is converted with phosphoryl chloride into a compound of the formula (VI) 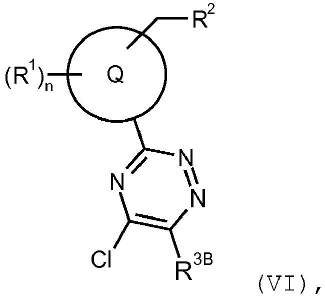 in which n, Q, R¹, R² and R^{3B} each have the meanings specified above,
and this is reacted directly with ammonia to give a compound of the formula (I-C) 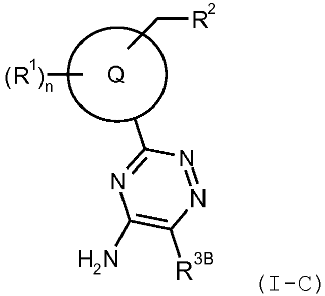 in which n, Q, R¹, R² and R^{3B} each have the meanings specified above,
and the resulting compounds of the formulae (I-A), (I-B) and (I-C) are, where appropriate, converted with the appropriate (i) solvents and/or (ii) acids or bases into their solvates, salts and/or solvates of the salts.

3. Compound of the formula (I) as defined in Claim 1 for the treatment and/or prophylaxis of diseases.

4. Use of a compound of the formula (I) as defined in Claim 1 for producing a medicament for the treatment and/or prophylaxis of heart failure, angina pectoris, hypertension, pulmonary hypertension, ischaemias, vascular disorders, renal insufficiency, thromboembolic disorders, fibrotic diseases and arteriosclerosis.

5. Medicament comprising a compound of the formula (I) as defined in Claim 1 in combination with an inert, nontoxic, pharmaceutically suitable excipient.

6. Medicament comprising a compound of the formula (I) as defined in Claim 1 in combination with a further active compound selected from the group consisting of organic nitrates, NO donors, cGMP-PDE inhibitors, antithrombotic agents, hypotensive agents and lipid metabolism modifiers.

7. Medicament according to Claim 5 or 6 for the treatment and/or prophylaxis of heart failure, angina pectoris, hypertension, pulmonary hypertension, ischaemias, vascular disorders, renal insufficiency, thromboembolic disorders, fibrotic disorders and arteriosclerosis.

## Revendications

1. Composé de formule (I) 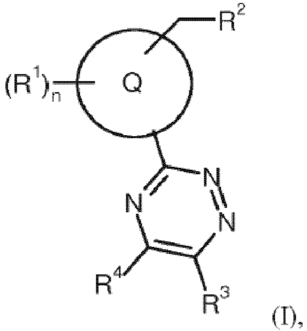 dans laquelle
le cycle Q représente un groupe de formule 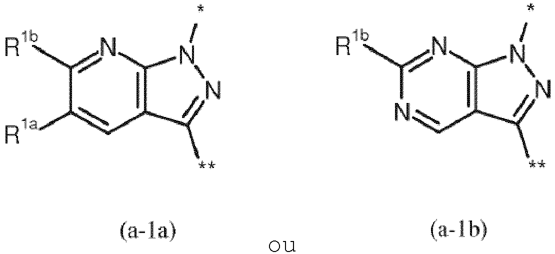 dans lesquelles
* représente l'emplacement de liaison à -CH₂-R²,
** représente l'emplacement de liaison au cycle triazine,
R^{1a} représente hydrogène ou fluor,
R^{1b} représente hydrogène ou méthyle,
R² représente 3,3,3-trifluoropro-1-yle, 2,2,3,3-tétrafluoroprop-1-yle, 2,2,3,3,3-pentafluoroprop-1-yle, phényle ou pyridyle,
le phényle étant substitué avec 1 à 3 substituants fluor,
et
le pyridyle pouvant être substitué avec 1 substituant fluor,
R³ représente difluorométhyle, trifluorométhyle, alkyle en (C₁-C₆), cyclopropyle, cyclobutyle, cyclopentyle, méthylsulfonylamino, méthoxycarbonylamino, phényle, pyrazolyle, oxazolyle ou pyridyle,
l'alkyle en (C₁-C₆) pouvant être substitué avec 1 à 3 substituants choisis indépendamment les uns des autres dans le groupe constitué par fluor, trifluorométhyle, cyclopropyle, cyclobutyle, cyclopentyle, difluorométhoxy, trifluorométhoxy, méthoxy et éthoxy,
et
le phényle, le pyrazolyle, l'oxazolyle et le pyridyle pouvant être substitués avec 1 ou 2 substituants choisis indépendamment l'un de l'autre dans le groupe constitué par fluor, chlore, difluorométhyle, trifluorométhyle, méthyle, éthyle, cyclopropyle, cyclobutyle, cyclopentyle, trifluorométhoxy, méthoxy et éthoxy,
R⁴ représente hydroxy ou amino,
ainsi que ses sels, solvates et solvates des sels.

2. Procédé de fabrication de composés de formule (I), tels que définis dans la revendication 1, **caractérisé en ce que**
[A] un composé de formule (II) 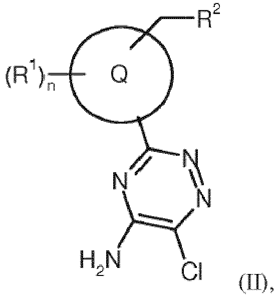 dans laquelle n, Q, R¹ et R² ont chacun les significations indiquées dans la revendication 1,
est mis en réaction dans un solvant inerte en présence d'un catalyseur de métal de transition approprié avec un composé de formule (III) 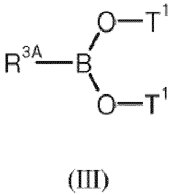 dans laquelle
R^{3A} représente phényle ou hétéroaryle à 5 ou 6 éléments,
le phényle et l'hétéroaryle à 5 ou 6 éléments pouvant être substitués avec 1 à 3 substituants choisis indépendamment les uns des autres dans le groupe constitué par halogène, trifluorométhyle, alkyle en (C₁-C₄), cycloalkyle en (C₃-C₇), difluorométhoxy, trifluorométhoxy et alcoxy en (C₁-C₆), et
T¹ représente hydrogène ou alkyle en (C₁-C₄), ou deux radicaux R¹¹ forment ensemble un pont -C(CH₃)₂-C(CH₃)₂, pour former un composé de formule (I-A) 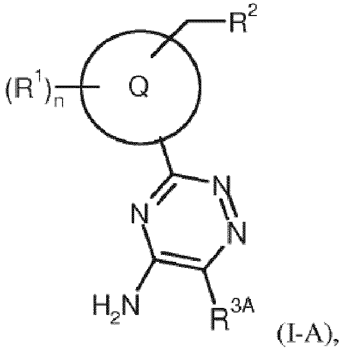 dans laquelle n, Q, R¹, R² et R^{3A} ont chacun les significations indiquées dans la revendication 1,
ou
[B] un composé de formule (IV) 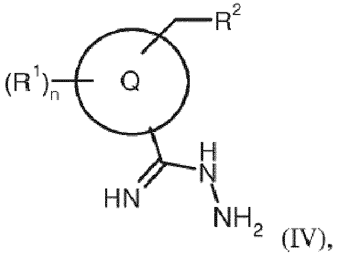 dans laquelle n, Q, R¹ et R² ont chacun les significations indiquées dans la revendication 1, est mis en réaction dans un solvant inerte avec un composé de formule (V) 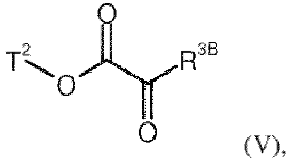 dans laquelle
R^{3B} représente difluorométhyle, trifluorométhyle, alkyle en (C₁-C₆) ou cycloalkyle en (C₃-C₇),
l'alkyle en (C₁-C₆) pouvant être substitué avec 1 à 3 substituants choisis indépendamment les uns des autres dans le groupe constitué par halogène, trifluorométhyle, alkyle en (C₁-C₄) , cycloalkyle en (C₃-C₇), difluorométhoxy, trifluorométhoxy et alcoxy en (C₁-C₆),
et
T² représente alkyle en (C₁-C₄),
pour former un composé de formule (I-B) 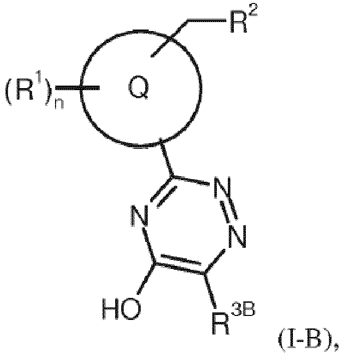 dans laquelle n, Q, R¹, R² et R^{3B} ont chacun les significations indiquées auparavant,
ou
[C] un composé de formule (I-B) est transformé avec du chlorure de phosphoryle en un composé de formule (VI) 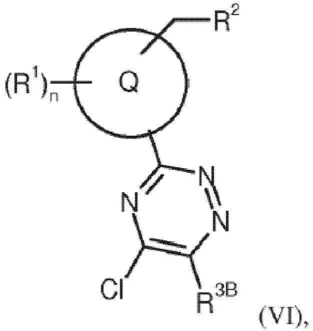 dans laquelle n, Q, R¹, R² et R^{3B} ont chacun les significations indiquées précédemment,
et celui-ci est directement mis en réaction avec de l'ammoniac pour former un composé de formule (I-C) 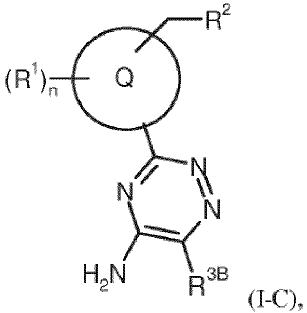 dans laquelle n, Q, R¹, R² et R^{3B} ont chacun les significations indiquées précédemment,
et les composés résultants de formule (I-A), (I-B) et (I-C) sont éventuellement transformés avec (i) les solvants et/ou (ii) les acides ou les bases appropriés en leurs solvates, sels et/ou solvates des sels.

3. Composé de formule (I), tel que défini dans la revendication 1, pour le traitement et/ou la prophylaxie de maladies.

4. Utilisation d'un composé de formule (I), tel que défini dans la revendication 1, pour le traitement et/ou la prophylaxie de l'insuffisance cardiaque, l'angine de poitrine, l'hypertension, l'hypertension pulmonaire, les ischémies, les maladies vasculaires, l'insuffisance rénale, les maladies thromboemboliques, les maladies fibrotiques et l'artériosclérose.

5. Médicament contenant un composé de formule (I), tel que défini dans la revendication 1, en combinaison avec un adjuvant inerte, non toxique, pharmaceutiquement approprié.

6. Médicament contenant un composé de formule (I), tel que défini dans la revendication 1, en combinaison avec un autre agent actif, choisi dans le groupe constitué par les nitrates organiques, les donneurs de NO, les inhibiteurs de cGMP-PDE, les agents antithrombotiques, les agents abaissant la tension artérielle, ainsi que les agents modifiant le métabolisme des lipides.

7. Médicament selon la revendication 5 ou 6, pour le traitement et/ou la prophylaxie de l'insuffisance cardiaque, l'angine de poitrine, l'hypertension, l'hypertension pulmonaire, les ischémies, les maladies vasculaires, l'insuffisance rénale, les maladies thromboemboliques, les maladies fibrotiques et l'artériosclérose.
